# EUROPEAN PATENT SPECIFICATION

(11) **EP 1 626 055 B1**
(45) Date of publication and mention of the grant of the patent: **05.12.2012**
(21) Application number: 05017405.1
(22) Date of filing: 10.08.2005
(51) Int. Cl.: C07K 7/06, C07K 7/23, A61K 38/09, C07K 16/26, C12N 15/00

(54) **Non-mammalian GnRH analogs and uses thereof in the regulation of the immune system**
Nicht von Säugetieren stammende GnRH Analoga und deren Verwendung zur Regulierung des Immunsystems
Analogues de GnRH non mammifère et leur utilisation pour la régulation du système immunitaire

(30) Priority: 10.08.2004 US 915553
(43) Date of publication of application: 15.02.2006
(73) Proprietor: Siler-Khodr, Theresa M., San Antonio, TX 78257 (US)
(72) Inventor: Siler-Khodr, Theresa M., San Antonio, TX 78257 (US)
(74) Representative: Walker, Ross Thomson

(56) References cited:
- WO-A-01/28576
- WO-A-01/74377
- WO-A-95/12309
- WO-A-03/016331
- WO-A-03/020205
- WO-A-2005/018660
- WO-A-2005/019448
- WO-A-2005/019457
- US-A- 5 168 061
- SILER-KHODR T M ET AL: "ACTION OF CHICKEN II GNRH ON THE HUMAN PLACENTA" JOURNAL OF CLINICAL ENDOCRINOLOGY AND METABOLISM, NEW YORK, NY, US, vol. 86, no. 2, February 2001 (2001-02), pages 804-810, XP000990838 ISSN: 0021-972X
- MILLAR ROBERT ET AL: "A novel mammalian receptor for the evolutionarily conserved type II GnRH" PROCEEDINGS OF THE NATIONAL ACADEMY OF SCIENCES OF USA, NATIONAL ACADEMY OF SCIENCE, WASHINGTON, DC, US, vol. 98, no. 17, 14 August 2001 (2001-08-14), pages 9636-9641, XP002178834 ISSN: 0027-8424
- BOYLE T A ET AL: "NUCLEOTIDE SEQUENCE ANALYSES PREDICT THAT HUMAN PITUITARY AND HUMAN PLACENTAL GONADOTROPIN-RELEASING HORMONE RECEPTORS HAVE IDENTICAL PRIMARY STRUCTURES" ENDOCRINE, MACMILLAN, BASINGSTOKE, GB, vol. 9, no. 3, December 1998 (1998-12), pages 281-287, XP008047328 ISSN: 1355-008X

### Sequence Listing

<110>Siler-Khodr, Theresa M.
<120>Non-Mammalian GnRH Analogs and Uses Thereof in the Immune System
<130>P7345.4
<140>US 10/915,553
   <141>2004-08-10
<160>16
<210>1
   <211>30
   <212>DNA
   <213>Gallus gallus (Chicken II GnRH)
<400>1
   cagcactggt cccatggctg gtaccctgga 30
<210>2
   <211>10
   <212>PRT
   <213>Unknown
<220>
   <221 >mat__peptide
   <222>unknown
   <223>Chicken II GnRH Analog. MOD_RES substitution of Gly residue at 10. Xaa at location 10 represents aza-Gly-NH2, ethylamide or other Gly amide. Xaa at location 6 represents D-Arg. MOD_RES Glu at position 1 is pyroglutamic acid.
<400>2
   Glu His Trp Ser His Xaa Trp Tyr Pro Xaa
   5 10
<210>3
   <211>30
   <212>DNA
   <213>Salmo salar (Salmon GnRH)
<400>3
   cagcactggt cttatggctg gctgcctgga 30
<210>4
   <211>10
   <212>PRT
   <213>Unknown
<220>
   <221>mat_peptide
   <222>unknown
   <223>Salmon GnRH Analog. MOD_RES substitution of Gly residue at 10. Xaa at location 10 represents aza-Gly-NH2, ethylamide or other Gly amide. Xaa at location 6 represents D-Arg. MOD_RES Glu at position 1 is pyroglutamic acid.
<400>4
   Glu His Trp Ser Tyr Xaa Trp Leu Pro Xaa
   5 10
<210>5
   <211>10
   <212>PRT
   <213>Homo sapiens
<220>
   <221 >mat_peptide
   <222>unknown
   <223>Mammalian GnRH. MOD_RES Glu at position 1 is pyroglutamic acid.
<400>5
   Glu His Trp Ser Tyr Gly Leu Arg Pro Gly
   5 10
<210>6
   <211>10
   <212>PRT
   <213>Gallus gallus
<220>
   <221>mat_peptide
   <222>unknown
   <223>Chicken II GnRH. MOD_RES Glu at position 1 is pyroglutamic acid.
<400>6
   Glu His Trp Ser His Gly Trp Tyr Pro Gly
   5 10
<210>7
   <211>10
   <212>PRT
   <213>Salmo salar
<220>
   <221>mat_peptide
   <222>unknown
   <223>Salmon GnRH. MOD_RES Glu at position 1 is pyroglutamic acid.
<400>7
   Glu His Trp Ser Tyr Gly Trp Leu Pro Gly
   5 10
<210>8
   <211>30
   <212>RNA
   <213>Gallus gallus (Chicken II GnRH)
<400>8
   gucgugacca ggguaccgac caugggaccu 30
   <210>9
   <211>30
   <212>RNA
   <213>Salmo salar (Salmon GnRH)
<400>9
   gucgugacca gaauaccgac cgacggaccu 30
<210>10
   <211>9
   <212>PRT
   <213>Artificial sequence
<220>
   <221>mat_peptide
   <222>unknown
   <223>Buserelin. MOD_RES Glu at position 1 is pyroglutamic acid.
   Xaa represents D-Ser (t-Bu). MOD_RES Pro residue at 9 bound to ethylamide.
<400>10
   Glu His Trp Ser Tyr Xaa Leu Arg Pro
   5
<210>11
   <211>9
   <212>PRT
   <213>Artificial sequence
<220>
   <221 >mat_peptide
   <222>unknown
   <223>Leuprolide. MOD_RES Glu at position 1 is pyroglutamic acid.
   Xaa represents D-Leu. MOD_RES Pro residue at 9 bound to ethylamide.
<400>11
   Glu His Trp Ser Tyr Xaa Leu Arg Pro
   5
<210>12
   <211>10
   <212>PRT
   <213>Artificial sequence
<220>
   <221 >mat_peptide
   <222>1,2,3,5,6,8,10
   <223>Antide. Xaa at location 1 is Ac-D-Nal, Xaa at location 2 is D-Cpa, Xaa at location 3 is D-Pal, Xaa at location 5 is NicLys, Xaa at location 6 is D-NicLys, Xaa at location 8 is ILys, Xaa at location 10 is D-Ala.
<400>12
   Xaa Xaa Xaa Ser Xaa Xaa Leu Xaa Pro Xaa
   5 10
<210>13
   <211>10
   <212>PRT
   <213>Gallus gallus
<220>
   <221 >mat_peptide
   <222>unknown
   <223>Chicken I GnRH. MOD_RES Glu at position 1 is pyroglutamic acid.
<400>13
   Glu His Trp Ser Tyr Gly Leu Gln Pro Gly
   5 10
<210>14
   <211>10
   <212>PRT
   <213>Lampetra genus
   <220>
   <221 >mat_peptide
   <222>unknown
   <223>Lamprey GnRH. MOD_RES Glu at position 1 is pyroglutamic acid.
<400>14
   Glu His Tyr Ser Leu Glu Trp Lys Pro Gly
   5 10
<210>15
   <211>30
   <212>DNA
   <213>Clupea harengus (Herring GnRH)
<400>15
   cagcactggt cttatggctg gctgcctgga 30
<210>16
   <211>10
   <212>PRT
   <213>Clupea harengus
<220>
   <221>mat_peptide
   <222>unknown
   <223>Herring GnRH Analog. MOD_RES substitution of Gly residue at 10 with aza-Gly-NH2, ethylamide or other Gly amide. Xaa represents D-Arg. MOD_RES Glu at position 1 is pyroglutamic acid.
<400>16
   Glu His Trp Ser Tyr Xaa Leu Ser Pro Gly
   5 10

### Sequence Listing

<110>Siler-Khodr, Theresa M.
<120>Non-Mammalian GnRH Analogs and Uses Thereof in the Immune System
<130>P7345.4
<140>US 10/915,553
   <141>2004-08-10
<160>16
<210>1
   <211>30
   <212>DNA
   <213>Gallus gallus (Chicken II GnRH)
<400>1
   cagcactggt cccatggctg gtaccctgga 30
<210>2
   <211>10
   <212>PRT
   <213>Unknown
<220>
   <221 >mat_peptide
   <222>unknown
   <223>Chicken II GnRH Analog. MOD_RES substitution of Gly residue at 10. Xaa at location 10 represents aza-Gly-NH2, ethylamide or other Gly amide. Xaa at location 6 represents D-Arg. MOD_RES Glu at position 1 is pyroglutamic acid.
<400>2
   Glu His Trp Ser His Xaa Trp Tyr Pro Xaa
   5 10
<210>3
   <211>30
   <212>DNA
   <213>Salmo salar (Salmon GnRH)
<400>3
   cagcactggt cttatggctg gctgcctgga 30
<210>4
   <211>10
   <212>PRT
   <213>Unknown
<220>
   <221 >mat_peptide
   <222>unknown
   <223>Salmon GnRH Analog. MOD_RES substitution of Gly residue at 10. Xaa at location 10 represents aza-Gly-NH2, ethylamide or other Gly amide. Xaa at location 6 represents D-Arg. MOD_RES Glu at position 1 is pyroglutamic acid.
<400>4
   Glu His Trp Ser Tyr Xaa Trp Leu Pro Xaa
   5 10
<210>5
   <211>10
   <212>PRT
   <213>Homo sapiens
<220>
   <221 >mat_peptide
   <222>unknown
   <223>Mammalian GnRH. MOD_RES Glu at position 1 is pyroglutamic acid.
<400>5
   Glu His Trp Ser Tyr Gly Leu Arg Pro Gly
   5 10
<210>6
   <211>10
   <212>PRT
   <213>Gallus gallus
<220>
   <221>mat_peptide
   <222>unknown
   <223>Chicken II GnRH. MOD_RES Glu at position 1 is pyroglutamic acid.
<400>6
   Glu His Trp Ser His Gly Trp Tyr Pro Gly
   5 10
<210>7
   <211>10
   <212>PRT
   <213>Salmo salar
<220>
   <221>mat_peptide
   <222>unknown
   <223>Salmon GnRH. MOD_RES Glu at position 1 is pyroglutamic acid.
<400>7
   Glu His Trp Ser Tyr Gly Trp Leu Pro Gly
   5 10
<210>8
   <211>30
   <212>RNA
   <213>Gallus gallus (Chicken II GnRH)
<400>8
   gucgugacca ggguaccgac caugggaccu 30
   <210>9
   <211>30
   <212>RNA
   <213>Salmo salar (Salmon GnRH)
<400>9
   gucgugacca gaauaccgac cgacggaccu 30
<210>10
   <211>9
   <212>PRT
   <213>Artificial sequence
<220>
   <221 >mat_peptide
   <222>unknown
   <223>Buserelin. MOD_RES Glu at position 1 is pyroglutamic acid.
   Xaa represents D-Ser (t-Bu). MOD_RES Pro residue at 9 bound to ethylamide.
<400>10
   Glu His Trp Ser Tyr Xaa Leu Arg Pro
   5
<210>11
   <211>9
   <212>PRT
   <213>Artificial sequence
<220>
   <221 >mat_peptide
   <222>unknown
   <223>Leuprolide. MOD_RES Glu at position 1 is pyroglutamic acid.
   Xaa represents D-Leu. MOD_RES Pro residue at 9 bound to ethylamide.
<400>11
   Glu His Trp Ser Tyr Xaa Leu Arg Pro
   5
<210>12
   <211>10
   <212>PRT
   <213>Artificial sequence
<220>
   <221 >mat_peptide
   <222>1,2,3,5,6,8,10
   <223>Antide. Xaa at location 1 is Ac-D-Nal, Xaa at location 2 is D-Cpa, Xaa at location 3 is D-Pal, Xaa at location 5 is NicLys, Xaa at location 6 is D-NicLys, Xaa at location 8 is ILys, Xaa at location 10 is D-Ala.
<400>12
   Xaa Xaa Xaa Ser Xaa Xaa Leu Xaa Pro Xaa
   5 10
<210>13
   <211>10
   <212>PRT
   <213>Gallus gallus
<220>
   <221 >mat_peptide
   <222>unknown
   <223>Chicken I GnRH. MOD_RES Glu at position 1 is pyroglutamic acid.
<400>13
   Glu His Trp Ser Tyr Gly Leu Gln Pro Gly
   5 10
<210>14
   <211>10
   <212>PRT
   <213>Lampetra genus
   <220>
   <221 >mat_peptide
   <222>unknown
   <223>Lamprey GnRH. MOD_RES Glu at position 1 is pyroglutamic acid.
<400>14
   Glu His Tyr Ser Leu Glu Trp Lys Pro Gly
   5 10
<210>15
   <211>30
   <212>DNA
   <213>Clupea harengus (Herring GnRH)
<400>15
   cagcactggt cttatggctg gctgcctgga 30
<210>16
   <211>10
   <212>PRT
   <213>Clupea harengus
<220>
   <221>mat_peptide
   <222>unknown
   <223>Herring GnRH Analog. MOD_RES substitution of Gly residue at 10 with aza-Gly-NH2, ethylamide or other Gly amide. Xaa represents D-Arg. MOD_RES Glu at position 1 is pyroglutamic acid.
<400>16
   Glu His Trp Ser Tyr Xaa Leu Ser Pro Gly
   5 10

## Description

### FIELD OF THE INVENTION

The present invention relates generally to the immune system. More particularly, it concerns the use of unique non-mammalian GnRH or its analogs or biometics designed to be useful in the immune system and with certain immune system disorders. Such disorders can include allergies and asthma, graft versus host disease, immune deficiency diseases, and autoimmune diseases, inflammatory responses, as well as immune processes regulating implantation and pregnancy and tumor rejection.

### BACKGROUND OF THE INVENTION

Before the chemical characterization of the mammalian hypothalamic GnRH (GnRH I)(SEQ ID NO: 5), it was realized that hypothalamic substances regulated production of pituitary LH and FSH. The delineation of GnRH I (SEQ ID NO: 5) has led to our understanding of its role in regulating pituitary LH. It also made possible the ability to create methods to detect and quantify this molecule. The human placenta and the chorionic membranes have also been observed to contain a GnRH-like substance. The present investigator has localized, quantified and demonstrated the synthesis of a GnRH-like substance by the human placenta. Burgus R., Guillemim R 1970 Hypothalamic releasing factors Ann Rev Biochem 39:499-526; Gibbons JM, Mitnick M, Chieffo V 1975 In vitro biosynthesis of TSH- and LH-releasing factors by the human placenta. Am J Obstet Gynecol 121:127-131; Siler-Khodr TM, Khodr GS 1978 Luteinizing hormone releasing factor content of the human placenta. Am J Obstet Gynecol 130:216-219; Khodr GS, Siler-Khodr TM 1978 Localization of luteinizing hormone releasing factor (LRF) in the human placenta. Fert Steril 29:523-526; Siler-Khodr TM, Khodr GS 1979 Extrahypothalamic luteinizing hormone releasing factor (LRF): Release of immunoreactive LRF by the human placenta in vitro. Fert Steril 22:294-296. Khodr GS, Siler-Khodr TM 1980 Placental LRF and its synthesis. Science 207:315-317.

The concentration of immunoreactive GnRH-like material in the placenta and maternal blood has been found to vary with gestational age, following a pattern similar to that of hCG. It was also demonstrated that exogenous GnRH I (SEQ ID NO: 5) can stimulate hCG production from human placental explants in vitro. Further, it was shown that the GnRH I (SEQ ID NO: 5) stimulation of hCG release was a receptor mediated event since it was specific and could be inhibited by a GnRH I antagonist, [N-Ac-Pro,D-p-Cl-Phe,D-Nal(2)]-GnRH I. In addition to the inhibition of hCG by this GnRH I antagonist, progesterone production was dramatically suppressed. The present investigator also observed that hCG response was related to the gestational age of the placenta. A gestational age-related action of the GnRH antagonist on the release of hCG and steroids was also observed. Further studies demonstrated a potent action of GnRH I (SEQ ID NO: 5) on placental prostanoids, again resulting in their inhibition when endogenous chorionic GnRH was the highest. The GnRH I antagonist also inhibited basal prostaglandin production with greater potency than equimolar concentrations of GnRH I (SEQ ID NO: 5) and this action was partially reversed by GnRH I (SEQ ID NO: 5). Chorionic GnRH was identified by the present investigator to regulate hCG in a paracrine fashion within the human placenta. These data demonstrated that this paracrine axis is of physiologic significance in cell to cell communication, and not of inconsequential, ectopic, tumor production. This was the first human paracrine system defined. Siler-Khodr TM, Khodr GS, Valenzuela G 1984 Immunoreactive gonadotropin-releasing hormone level in maternal circulation throughout pregnancy. Am J Obstet Gynecol 150:376-379; Sorem KA, Smirkle CB, Spencer DK, Yoder BA, Grayson MA, Siler-Khodr TM 1996 Circulating maternal CRH and GnRH in normal and abnormal pregnancies. Am J Obstet Gynecol 175:912-916; Khodr GS, Siler-Khodr TM 1979 The effect of luteinizing hormone releasing factor (LRF) on hCG secretion Fert Steril 30:301-304; Siler-Khodr TM, Khodr GS 1981 Dose response analysis of GnRH stimulation of hCG releases from human term placenta. Biol Reprod 25:353-358; Siler-Khodr TM, Khodr GS 1979 Extrahypothalamic luteinizing hormone releasing factor (LRF): Release of immunoreactive LRF by the human placenta in vitro. Fert Steril 22:294-296; Siler-Khodr TM, Khodr GS, Vickery BH, Nestor JJ, Jr. 1983 Inhibition of hCG, alpha hCG and progesterone release from human placental tissue in vitro by a GnRH antagonist. Life Sci 32:2741-2745; Siler-Khodr TM, Khodr GS, Valenzuela G, Rhode J 1986 Gonadotropin-releasing hormone effects on placental hormones during gestation: 1 Alpha-human chorionic gonadotropin, human chorionic gonadotropin and human chorionic somatomammotropin. Biol Reprod 34:245-254; Siler-Khodr TM, Khodr GS, Rhode J, Vickery BH, Nestor JJ, Jr. 1987 Gestational age related inhibition of placental hCG, hCG and steroid hormone release in vitro by a GnRH antagonist. Placenta 8:1-14; Siler-Khodr TM, Khodr GS, Valenzuela G, Harper J, Rhode J 1986 GnRH effects on placental hormones during gestation. 111 Prostaglandin E, prostaglandin F, and 13, 14-dihydro-15-keto-prostaglandin F. Biol Reprod 35:312-319; Kang IS, Koong MY, Forman JS, Siler-Khodr TM 1991 Dose-related action of GnRH on basal prostanoid production from the human term placenta. Am J Obstet Gynecol 165:1771-1777; Siler-Khodr, M, Khodr GS, Harper MJ, Rhode J, Vickery BH, Nestor JJ, Jr. 1986 Differential inhibition of human placental prostaglandin release in vitro by a GnRH antagonist. Prostaglandins 31:1003-1010; Siler-Khodr T.M. and G.S. Khodr. 1981. The production and activity of placental releasing hormones. In Fetal Endocrinology. J. Resko and W. Montagna, editors. Academic Press Inc. New York. 183-210; Siler-Khodr, T.M. and G.S. Khodr. 1982 GnRH in the placenta. In role of Peptides and Proteins in Control of Reproduction; D.S. Khindsa and S.M. McCann, editors. Elsevier North Holland, New York. 347-363; Siler-Khodr TM 1983 Hypothalamic-like releasing hormones of the placenta. Clin Perinatol 10:533-566; Siler-Khodr TM 1983 Hypothalamic-like peptides of the placenta. Semin Reprod Endocrinol 1:321-333.

The isolation and characterization of a GnRH I (SEQ ID NO: 5) gene in the placenta, which is transcribed to a mRNA identical to that in the hypothalamus with the exception of the inclusion of the first intron and a very long first exon, has been reported. The message has been localized to the syncytio- and cytotrophoblast, as well as the stroma of the placenta, and is present in higher concentrations during the first half of pregnancy. Multiple transcription sites have been identified for the GnRH I (SEQ ID NO: 5) gene in reproductive tissues, including the placenta. Further, steroid regulatory sites on the promoter have also been identified. The functionality of this promoter is supported by showing that GnRH I mRNA can be regulated by steroids. Dong KW, Yu KL, Roberts JL 1993 Identification of a major up-stream transcription start site for the human pro gonadotropin-releasing hormone gene used in reproductive tissues and cell lines. Chandran UR, Attardi B, Friedman R, Dong KW, Roberts JL, DeFranco DB 1994 Glucocorticoid receptor-mediated repression of gonadotropin-releasing hormone promoter activity in GTI hypothalamic cell lines. Endocrinology 134:1467-1474; Dong KW, Chen ZG, Cheng KW, Yu KL 1996 Evidence for estrogen receptor-mediated regulation of human gonadotropin-releasing hormone promoter activity in human placental cells. Mol Cell Endocrinol 117:241-246; Mol Endocrinol 7:1654-166; Dong KW, Duval P, Zeng Z, Gordon K, Williams RF, Hodgen GD, Jones G, Kerdelhue B, Roberts JL 1996 Multiple transcription start sites for the GnRH gene in rhesus and cynomolgus monkeys: a non-human primate model for studying GnRH gene regulation. Mol Cell Endocrinol 117:121-130; Dong KW, Yu KL, Chen ZG, Chen YD, Roberts JL 1997 Characterization of multiple promoters directing tissue-specific expression of the human gonadotropin-releasing hormone gene. Endocrinology 138:2754-2762; Duello TM, Tsai SJ, Van Ess PJ 1993 In situ demonstration and characterization of pro gonadotropin-releasing hormone messenger ribonucleic acid in first trimester human placentas. Endocrinology 133:2617-262-3; Kelly AC, Rodgers A, Dong KW, Barrezueta NX, Blum M, Roberts JL 1991 Gonadotropin-releasing hormone and chorionic gonadotropin gene expression in human placental development DNA Cell Biol 10:411-421; Radovick S, Wondisford FE, Nakayama Y, Yamada M, Cutler GB, Jr., Weintraub BD 1990 Isolation and characterization of the human gonadotropin-releasing hormone gene in the hypothalamus and placenta. Mol Endocrinol 4:476-480; Adelman JP, Mason AJ, Hayflick JS, Seeburg PH 1986 Isolation of the gene and hypothalamic cDNA for the common precursor of gonadotropin-releasing hormone and prolactin release-inhibiting factor in human and rat. Proc Natl Acad Sci U S A 83:179-183; Seebirg PH, Adelman JP 1984 Characterization of cDNA for precursor of human luteinizing hormone releasing hormone. Nature 311:666-668; Joss JM, King JA, Millar RP 1994 Identification of the molecular forms of and steroid hormone response to gonadotropin-releasing hormone in the Australian lungfish Neoceratodus forsteri. Gen Comp Endocrinol 96:392-400; Montero M, Le Belle N, King JA, Millar RP, Dufour S 1995 Differential regulation of the two forms of gonadotropin-releasing hormone (mGnRH and chicken GnRH-II) by sex steroids in the European female silver eel (Anguilla anguilla). Neuroendocrinology 61:525-535; Ikeda M, Taga M, Sakakibara H, Minaguchi H, Ginsburg E, Vonderhaar BK 1996 Gene expression of gonadotropin-releasing hormone in early pregnant rat and steroid hormone exposed mouse uteri. J Endocrinol Invest 19:708-713; Gothilf Y, Meiri I, Elizur A, Zohar Y 1997 Preovulatory changes in the levels of three gonadotropin-releasing hormone-encoding messenger ribonucleic acids (mRNAs), gonadotropin. B-subunit mRNAs plasma gonadotropin, and steroids in the female gilthead seabream, Sparus aurata, Biol Reprod 57:1145-1154.

The GnRH I (SEQ ID NO: 5) receptor in the placenta has not been characterized as fully as the GnRH I (SEQ ID NO: 5) receptor in the pituitary. A placental GnRH receptor having a Ka of only 10⁻⁶M for GnRH I (SEQ ID NO: 5) has been reported. In addition, superagonist or antagonist for the pituitary GnRH receptor show very different affinity for the placental GnRH receptor. Sealfon SC, Weinstein H, Millar RP 1997 Molecular mechanism of ligand interaction with the gonadotropin-releasing hormone receptor. Endocr Rev 18:180-205; Karten MJ, Rivier JE 1986 Gonadotropin-releasing hormone analog design. Structure-function studies toward the development of agonists and antagonists: Rationale and perspective. Endocr Rev 7:44-66. Escher E, Mackiewicz Z, Lagace G, Lehoux J, Gallo-Payet N, Bellabarba D, Belisle S 1988 Human placental LHRH receptor: Agonist and antagonist labeling produces differences in the size of the non-denatured, solubilize receptor; J Recept Res 8:391-405; Bramley TA, McPhie CA, Menzies GS 1992 Human placental gonadotropin-releasing hormone (GnRH) binding sites: Characterization, properties and ligand specificity. Placenta 12:555-581.

GnRH receptor activity, as well as the mRNA for the GnRH I receptor, vary throughout gestation in the human placenta. This receptor is greatest in early gestation and appears to be down regulated by 12-20 weeks. While the receptor is again detectable in term placentas, the mRNA (using a GnRH I decapeptide probe and in situ hybridization methodology) was undetectable at this state of gestation. This pattern of receptor activity is consistent with the concentration of GnRH-like material in placental tissue and maternal blood throughout gestation, and supports the hypothesis that chorionic GnRH may down-regulate its chorionic receptors, as can GnRH I (SEQ ID NO: 5), and its analogs at the pituitary level. Studies by the present investigator and those of Barnea et al, have demonstrated competitive inhibition by GnRH I antagonist. Other studies of Szilagyi et al. and Currie et al. indicate that GnRHI agonist can down-regulate the placental GnRH receptor. In addition, the demonstration that the placental GnRH receptor can be up regulated in cell cultures by estradiol supports the hypothesis that this receptor is functional in the regulation of placental hormonogenesis. Bramley TA, McPhie CA, Menzies GS 1994 Human placental gonadotropin-releasing hormone (GnRH) binding sites: 111. Changes in GnRH binding levels with stage of gestation. Placenta 15:733-745; Lin LS, Roberts VJ, Yen SS 1997 Expression of human gonadotropin-releasing hormone receptor gene in the placenta and its functional relationship to human chorionic gonadotropin secretion. J Clin Endocrinol Metab 80:580-585; Bramley TA, McPhie CA, Menzies GS 1994 Human placental gonadotropin-releasing hormone (GnRH) binding sites: 111. Changes in GnRH binding levels with stage of gestation. Placenta 15:733-745; Lin LS, Roberts VJ, Yen SS 1997 Expression of human gonadotropin-releasing hormone receptor gene in the placenta and its functional relationship to human chorionic gonadotropin secretion. J Clin Endocrinol Metab. 80: 580-585 Siler-Khodr TM, Khodr GS, Valenzuela G 1984 Immunoreactive gonadotropin-releasing hormone level in maternal circulation throughout pregnancy. Am J Obstet Gynecol 150:376-379; Siler-Khodr TM, Khodr GS 1978 Luteinizing hormone releasing factor content of the human placenta. Am J Obstet Gynecol 130:216-219; Siler-Khodr TM, Khodr GS, Vickery BH, Nestor JJ, Jr. 1983 Inhibition of hCG, alpha hCG and progesterone release from human placental tissue in vitro by a GnRH antagonist. Life Sci 32:2741-2745; Siler-Khodr TM, Khodr GS, Harper MJ, Rhode J, Vickery BH, Nestor JJ, Jr. 1986 Differential inhibition of human placental prostaglandin release in vitro by a GnRH antagonist. Prostaglandins 31:1003-1010; Barnea ER, Kaplan M, Naor Z 1991 Comparative stimulatory effect of gonadotropin releasing hormone (GnRH) and GnRH agonist upon pulsatile human chorionic gonadotropin secretion in superfused placental explants: reversible inhibition by a GnRH antagonist. Hum Reprod 6:1063-1069; Szilagyi A, Benz R, Rossmanith WG 1992 The human first-term placenta in vitro: regulation of hCG secretion by GnRH and its antagonist. Gynecol Endocrinol 6:293-300; Currie WD, Setoyarna T, Lee PS, Baimbridge KG, Church J, Yuen BH, Leung PC 1993 Cytosolic free Ca2+ in human syncytiotrophoblast cells increased by gonadotropin-releasing hormone. Endocrinology 133:2220-2226; Barnea ER, Kaplan M, Naor Z 1991 Comparative stimulatory effect of gonadotropin releasing hormone (GnRH) and GnRH agonist upon pulsatile human chorionic gonadotropin secretion in superfused placental explants: reversible inhibition by a GnRH antagonist. Hum Reprod 6:1063-1069; Bliatacharya S, Chaudhary J, Das C 1992 Responsiveness to gonadotropin releasing hormone of human term trophoblast cells in vitro: induction by estradiol. Biochem Int 28:363-371.

Another factor that regulates a hormone's activity is its metabolism. The enzyme that degrades GaRH I differs during pregnancy from the enzyme that degrades GnRH I in the degrades GnRH I differs during pregnancy from the enzyme that degrades GnRHI in the pituitary or the blood of non-pregnant individuals. In placental tissue, the primary enzymatic activity for the degradation of GnRH I (SEQ ID, NO:5) is chorionic peptidase-1 (C-ase-1), a post-proline peptidase. C-ase-1 is a glycoprotein with a molecular weight of 60,000. It acts as a post-proline peptidase, and is inhibited by bacitracin, para-amino-benzamidine, acetopyruvate and certain cations. GnRH I (SEQ ID NO: 5) is actively degraded by C-ase-1 at neutral pH, having a Km of 10⁻⁸M. Using immunofluorescent methodology, C-ase-1 has been localized by the present inventor in the cytoplasm of the syncytiotrophoblast and syncytial buds. It is secreted into material blood, where GnRH I (SEQ. ID NO: 5) is not stable without specific inhibitors of this post-proline peptidase. G-ase-1 is present in very high concentrations, and accounts for most of the GnRH (SEQ ID NO: 15) degradation activity in the placenta under physiological conditions. Siler-Khodr TWI, Kang IS, Jones MA, Harper MJK Khodr GS, Rhode J 1989. Characterization and purification of a placental protein that inactivates GnRH, TRH and Angiotensin 11. Placenta 10:283-296; Kang IS, Siler-Khodr TM 1992 Chorionic peptidase inactivates GnRH as a post-proline peptidase. Placenta 13:81-87; Siler-Khodr Two, Kang IS, Jones MA, Harper MJK, Khodr GS, Rhode J 1989 Characterization, and purification of a placental protein that inactivates GnRH, TRH and Angiotensin 11. Placenta 10:283-296; Kang IS, Gallwitz J, Guzman V, Siler-Khodr TM 1990 Definition of the enzyme kinetics and optimal activity of chorionic peptidase-1. The 23rd Annual Meeting of the Society for the Study of Reproduction (Vancouver) (Abstract #311):144(Abstr.). Benuck M, MarkaN 1976 Differences in the degradation of hypothalamic releasing factors by rat and human serum. Life Sci 19:1271-1276.

These *in vitro* studies support the hypothesis of the specific, receptor-mediated and enzyme-regulated action of GnRH I (SEQ ID NO: 5) on placental hormonogenesis and demonstrate the paracrine effects and feedback interactions for numerous intrauterine hormones interacting with chorionic GnRH. They formed the basis for studies of such axes in other tissues such as the immune system.

Petraglia et al have described the pulsatile release of a GnRH-like substance, which has a specific pulse frequency, amplitude and duration, with increased amplitude during early gestation. Further studies on the action of GnRH I (SEQ ID NO: 5) and its analogs *in vivo* have demonstrated these paracrine interactions for chorionic GnRH-like activity and numerous other chorionic hormones and established the physiologic role of GnRH in the maintenance of normal pregnancy. The secretion of a GnRH-like substance by the peri-implantation rhesus monkey embryo, which precedes the secretion of chorionic gonadotropin, has been demonstrated. Other investigators have shown that administration of high doses of GnRH I (SEQ ID NO: 5), its agonistic analogs or antibodies, to pregnant baboons and monkeys effects a sharp decrease of CG production and progesterone, which in most cases leads to termination of pregnancy. Interruption of pregnancy was most consistently observed when these GnRH I analogs were administered around the time of or shortly following implantation. We have administered GnRH I antagonists to pregnant baboons of more than 35 days post implantation and in some animals we observed pregnancy loss or a negative outcome of pregnancies. In a saline controlled study we administered GnRH I agonist or an antagonist to pregnant baboon just following implantation and have found pregnancy loss using high doses in many of these animals. Petraglia F, Genazzani AD, Aguzzoli L, Gallinelli A, de Vita D, Caruso A, Genazzani AR 1994 Pulsatile fluctuations of plasma-gonadotropin-releasing hormone and corticotropin-releasing factor levels in healthy pregnant women. Acta Obstet Gynecol Scand 73:284-289; Siler-Khodr, T.M. 1993. Luteinizing Hormone Releasing Hormone (LHRH) and the Placenta and Fetal Membranes. In Molecular Aspects of Placental and Fetal Membrane Autocoids. G.E. Rice and S.P. Brennecke, editors. CRC Press, Inc. Ann Arbor. 339-350; Petraglia F, Calza L, Garuti GC, Giardino L, De Ramundo BM, Angioni S 1990 New aspects of placental endocrinology. J Endocrinol Invest 65:262-267; Seshagiri PB, Terasawa E, Heam JP 1994 The secretion of gonadotropin-releasing hormone by peri-implantation embryos of the rhesus monkey: comparison with the secretion of chorionic gonadotropin. Hum Reprod 9:1300-1307; Gupta SK, Singh M 1985 Characteristics and bioefficacy of monoclonal antigonadotropin releasing hormone antibody. Am J. Repro Immunol Microbiol 7:104-108; Das C, Gupta SK, Talwar GP 1985 Pregnancy interfering action of LHRH and anti-LHRH. J. Steroid Biochem 23:803-806; Hodges JK, Hearn JP 1977 Effects of immunization against luteinizing hormone releasing hormone on reproduction of the marmoset monkey Callithrix jacchus. Nature 265:746-748; Vickery BH, McRae GI, Stevens VC 1981 Suppression of luteal and placental function in pregnant baboons with agonist analogs of luteinizing hormone-releasing hormones. Fert Steril 36:664-668; Das C, Talwar GP 1983 Pregnancy-terminating action of a luteinizing hormone-releasing hormone agonist D-Ser(But)6desGlylOProEA in baboons. Fert Steril 39:218-223; Rao A, Moudgal N 1984 Effect of LHRH injection on serum chorionic: gonadotropin levels in the pregnant bonnet monkey (Macaca radiata). Obstet Gynecol 12:1105-1106; Rao AJ, Chakraborti R, Kotagi SG, Ravindranath N, Moudgal NR 1985 Effect of LHRH agonists and antagonists in male and female bonnet monkeys (Macaca Radiata). J. Steroid Biochem 23:807-809; T. M. Siler-Khodr, T. J. Kuehl, and B. H. Vickery. Effects of a gonadotropin-releasing hormone antagonist on hormonal levels in the pregnant baboon and on fetal outcome. Fertil.Steril. 41:448-454, 1984; T.M. Siler-Khodr, T. Kuehl, and B. Vickery. Action ofa GnRH antagonist on the pregnant baboon. The 32nd Annual Meeting of The Society for Gynecologic Investigation (Phoenix) (Abstract #249):142, 1985. (Abstract); I. S. Kang, T. J. Kuehl, and T. M. Siler-Khodr. Effect of treatment with gonadotropin-releasing hormone analogues on pregnancy outcome in the baboon. Fertil.Steril. 52:846-853, 1989.

In pregnant women, administration of low doses of GnRH I (SEQ ID NO: 5) does not significantly change circulating hCG. However, this finding was dose and gestational age related. A recent study of Devreker et.al. reports that the use of long-acting,GnRH analogs in IVF impaired the implantation rate. While these GnRH I analogs have proven to be generally nontoxic, long-term chronic use has been associated with a hypo-estrogenic state. Accidental administration of GnRH analogs during early pregnancy has been reported with varied outcomes. Generally, pregnancy outcomes appeared unaffected, but increased cases of spontaneous abortion and pre-term labor have also been observed. The varied outcomes may reflect the different doses and protocols of administration of these GnRH I analogs as well as the: different analogs employed. For analogs that can be rapidly metabolized by the chorionic tissues, little effect, if any, would be anticipated In addition, the affinity for the placental receptor for many of these GnRH I analogs is greatly reduced as compared to the pituitary receptors affinity and they are degraded by the placental enzymes. In those cases, little chorionic effect would be observed. Tamada T, Akabori A, Konuma S, Araki S 1976 Lack of release of human chorionic. gonadotropin by gonadotropin-releasing hormone. Endocrinol Jap 23:531-533; Perez-Lopez FR, Robert J, Teijeiro J 1984 Prl, TSH, FSH, B-hCG and oestriol responses to repetitive (triple) LRH/TRH administration in the third trimester of human pregnancy. Acta Endocrinol 106:400-404; Egyed J, Gati I 1985 Elevated serum hCG level after intravenous LH-RH administration in human pregnancies. Endocrinol Exp 19:11-15; Iwashita M, Kudo Y, Shinozaki Y, Takeda Y 1993 Gonadotropin-releasing hormone increases serum human chorionic gonadotropin in pregnant women. Endocrine Journal 40:539-544; Devreker F, Govaerts I, Bertrand E, Van den Bergh M, Gervy C, Englert Y 1996 The long-acting gonadotropin-releasing hormone analogues impaired the implantation rate. Fert Steril 65:122-126; Siler-Khodr, T.M. 1994. Potentials for embryo damage of GnRH analogs. In Ovulation Induction: Basic Science and Clinical Advances. M. Filicor and C. Flamigni, editors Elsevier Science B.V. Amsterdam. 279-306.

The ovaries are also known to produce a GnRH-like peptide. The presence of a GnRH receptor was first described in rat luteal cells in 1979. A GnRH receptor in human corpus luteum was later described by Bramley et al and the expression of a mRNA for GnRH I (SEQ ID NO: 5) in human ovarian tissues was later described by others. However, the affinity of the ovarian and placental receptor for GnRH I (SEQ ID NO: 5) or its analogs is greatly reduced as compared to the pituitary's receptor for GnRH I (SEQ ID NO: 5). Other investigators have described GnRH I mRNA expression in the fallopian tube and the early embryo. In addition, the expression of mRNA for GnRH I (SEQ ID NO: 5) in the endometrium has been reported. Aten RF, Williams AT, Behrman HR. Ovarian gonadotropin-releasing hormone-like protein(s): demonstration and characterization. Endocrinology 1986; 118: 961-967; Aten RF, Polan ML, Bayless R, Behrman HR. A gonadotropin-releasing hormone (GnRH)-like protein in human ovaries: similarity to the GnRH-like ovarian protein of the rat. J. Clin. Endocrinol. Metab. 1987; 64: 1288-1293; Peng C, Fan NC, Ligier M, Vaananen J, Leung PCK. Expression and regulation of gonadotropin-releasing hormone (GnRH) and GnRH receptor messenger ribonucleic acids in human granulosa-luteal cells. Endocrinology 1994; 135: 1740-1746; Clayton RN, Harwood JP, Catt KJ. Gonadotropin-releasing hormone analogue binds to luteal cells and inhibits progesterone production. Nature 1979; 90: 282; Bramley TA, Menzies GS, Baird DT. Specific binding of gonadotropin-releasing hormoneand an agonist to human corpus luteum homogenates: Characterization, properties, and luteal phase levels. J. Clin. Endocrinol. Metab. 1985; 61: 834-841; Dong KW, Yu KL, Roberts JL. Identification of a major up-stream transcription start site for the human progonadotropin-releasing hormone gene used in reproductive tissues and cell lines. Mol. Endocrinol. 1993; 7: 1654-1666; Casan EM, Raga F, Polan ML. GnRH mRNA and protein expression in human preimplantation embryos. Mol. Hum. Reprod. 1999; 5: 234-239; Casan EM, Raga F, Bonilla-Musoles F, Polan ML. Human oviductal gonadotropin-releasing hormone: possible implications in fertilization, early embryonic development, and implantation. J. Clin. Endocrinol. Metab. 2000; 85: 1377-1381; Raga F, Casan EM, Kruessel JS, Wen Y, Huang H-Y, Nezhat C, Polan ML. Quantitative gonadotropin-releasing hormone (GnRH) gene expression and immunohistochemical localization in human endometrium throughout the menstrual cycle. Biol. Reprod. 1998; 59: 661-669; Casan EM, Raga, Kruessel JS, et.al. Immunoreactive gonadotropin-releasing hormone expression in the cycling human endometrium of fertile patients. Fertil. Steril. 1998; 70: 102-106.

Most studies describing the activity of GnRH I (SEQ ID NO: 5) and its analogs in extra-pituitary tissues have been plagued with the finding of low affinity binding sites and the high concentration of GnRH I (SEQ ID NO: 5) required to affect tissue function. Similar observations have been made for the placental GnRH receptor. Although there is substantial data to support the hypothesis that there are active GnRH axes in the ovary, endometrium and the placenta, the physiologic relevance of GnRH I (SEQ ID NO: 5) in these extra-pituitary reproductive tissues seemed questionable. Bramley TA, McPhie CA, Menzies GS. Human placental gonadotrophin-releasing hormone (GnRH) binding sites: I. Characterization, properties and ligand specificity. Placenta 1992; 13: 555-581.

It has previously been accepted that only non-mammalian vertebrates have multiple forms of GnRH in the same species. Therefore, the hypothesis of more than one form of GnRH in the human placenta was considered dubious. Yet, the reduced affinity of the chorionic receptor for GnRH I (SEQ ID NO: 5), the biphasic activity of GnRH I (SEQ ID NO: 5) on the human placental hormonogenesis and our observation of immunological differences in the chorionic GnRH, led us to consider the possibility that the human may express another isoform of GnRH. In 1993, Dellovad, et al. and in 1994, King et al. described chicken II GnRH (GnRH II) (SEQ ID NO: 6) in shrew, mole and bat brain, thus demonstrating that two different isomers of GnRH existed in the mammal brain. GnRH II (SEQ ID NO: 6) has now been characterized in the guinea pig and in the human brain. Separate genes for GnRH II (SEQ ID NO: 6) and GnRH I (SEQ ID NO: 5) have also been described. Dellovade TL, King JA, Millar RP, Rissman EF 1993 Presence and differential distribution of distinct forms of immunoreactive gonadotropin-releasing hormone in the musk shrew brain. Neuroendocrinology 58:166-177; King JA, Steneveld A A, Curlewis JD, Rissman EF, Millar RP 1994 Identification of chicken GnRH II in brains of inetatherian and early-evolved eutherian species of mammals. Regul Pept 54:467-477; Jimenez-Linan M, Rubin BS, King JC 1997 Examination of guinea pig luteinizing hormone-releasing hormone gene reveals a unique decapeptide and existence of two transcripts in the brain. Endocrinology 13 8:4123-4130; Lescheid D, Terasawa E, Abler LA, Urbanski HF, Warby CM, Millar RP, Sherwood NM 1997 A second form of gonadotropin-releasing hormone (GnRH) with characteristics of chicken GnRH- II is present in the primate brain. Endocrinology 138:1997; White SA, Bond CT, Francis RC, Kasten TL, Fernald RD, Adelman JP 1994 A second gene for gonadotropin-releasing hormone: cDNA and expression pattern in the brain. Proc Natl Acad Sci U S A 91:1423-1427; Lin XW, Peter RE 1997 Cloning and expression pattern of a second [His5Trp7Tyr8] gonadotropin-releasing hormone (chicken GnRH-H-11) mRNA in goldfish; evidence for two distinct genes. Gen Comp Endocrinol 107:262-272.

Other isomers of GnRH, such as salmon GnRH (SEQ ID NO: 7) and GnRH II (SEQ ID NO: 6), have a higher affinity for the placental GnRH receptor, yet bind with a lesser affinity to the human pituitary GnRH receptor. These data demonstrate, together with our prior findings, the existence of a specific placental receptor for GnRH-like molecules, and another GnRH ligand for this receptor. In amphibians, a GnRH II (SEQ ID NO: 6) receptor as well as a mammalian GnRH (SEQ ID NO: 5) receptor have been shown. The specificity and evolutionary aspects of the GnRH receptor have been studied in many species. GnRH I (SEQ ID NO: 5) has been reported to be active in many vertebrate classes. Other GnRHs, such as GnRH II (SEQ ID NO: 6) and salmon GnRH (SEQ ID NO: 7), have reduced affinity for the mammalian pituitary GnRH I receptor. R. P. Millar, R. C. Milton, B. K. Follett, and J. A. King. Receptor binding and gonadotropin-releasing activity of a novel chicken gonadotropin-releasing hormone ([His5, Trp7, Tyr8]GnRH) and a D-Arg6 analog. Endocrinology 119 (1):224-231, 1986; K. Miyamoto, Y. Hasegawa, M. Nomura, M. Igarashi, K. Kangawa, and H. Matsuo. Identification of the second gonadotropin-releasing hormone in chicken hypothalamus: evidence that gonadotropin secretion is probably controlled by two distinct gonadotropin-releasing hormones in avian species. Proceedings of the National Academy of Sciences of the United States of America 81 (12):3874-3878, 1984; Bramley TA, McPhie CA, Menzies GS 1992 Human placental gonadotropin-releasing hormone (GnRH) binding sites: Characterization, properties and ligand specificity. Placenta 12:555-581.

These prior data led us to investigate the possibility that other GnRH isoforms are active in the extra-pituitary tissues, and bind to ovarian, tubal and/or uterine GnRH receptors with high affinity and have enhanced bioactivity on the regulation of these tissue functions. Thus, our continued studies focused on the activities of non-mammalian analogs and isoforms of GnRH on extra-pituitary tissue regulation. We have demonstrated the localization and production of GnRH II (SEQ ID NO: 6) by the human placenta and have shown the presence of a specific receptor for this GnRH II (SEQ ID NO: 6) and the biologic activity of GnRH II (SEQ ID NO: 6) and its stable analogs on human placental hormonal production. T. M. Siler-Khodr and M. Grayson. Action of chicken II GnRH on the human placenta. J.Clin.Endocrinol.Metab. 86:804-810,2001; T. M. Siler-Khodr and M. Grayson. Salmon GnRH and its analogs bind the human placental receptor. J.Soc.Gynecol.Invest. 8:233-238, 2001.

We have performed similar studies using primate and human ovarian, fallopian tube and uterine tissues and have demonstrated a high affinity specific GnRH II (SEQ ID NO: 6) receptor and bioactivity in these tissues without significant action on pituitary LH. Studies in vivo have demonstrated that chronic administration of long-acting GnRH II (SEQ ID NO: 6) and its stable analogs to rhesus monkeys is are effective contraceptive agent, which does not inhibit regular hypothalamic-pituitary-gonadal driven menstrual cycle. T. M. Siler-khdor, M. Grayson, C. A. Eddy. Action of gonadotropin.releasing hormone II on the baboon ovary. Biol.Reprod. 68:1150-1156, 2003.

WO03/02205 A discloses GnRH analog decapeptides resistant to degradation by the placental enzyme C-ase-1 or a post-proline peptidase.

The present Applicant has found that the non-mammalian GnRH analogs (SEQ. ID NO: 2 and SEQ ID NO: 4) are also useful in the immune system and in various immune system disorders. We have performed a number of studies on GnRH II (SEQ. ID NO: 6) in the immune system, relating to its localization and that of its specific receptor and its effect on B cells, monocytes, macrophages, dendritic cell and natural killer cells and immune system cells and functions. It has been reported that GnRH II (SEQ ID NO: 6) stimulates T cells adhesion and homing, but these effects were only seen after- twenty--four hours. Thus, this effect appears to be secondary to a more immediate action. Based on our studies of GnRH H (SEQ ID NO: 6) and its specific receptor localization and its activity on leukocytes, we propose that GnRH II (SEQ ID NO: 6) and our analogs (SEQ ID NO: 2 and SEQ ID NO: 4) effect monocytes, macrophages, B cells, dendritic cells, mast and natural killer cells directly. Other than ours, no studies on specifically designed stable GnRH II receptor analogs have been reported. Our studies of GnRH II (SEQ ID NO: 6), its analog (SEQ ID NO: 2) and its specific receptor have led to our proposal that GnRH II (SEQ ID NO: 6) regulates cells of the immune system, including but not limited to monocyte and macrophage, B cell, dendritic cells, mast and naturalkiller cells differentiation and function. These GnRH II receptor mediated events participate in the regulation of what is recognized to be foreign - be it a sperm, implantation, and endometrial implant, tumor acceptance, another self protein, tissue transplantation, tumor rejection or an infection such as a

virus, such as HIV, and form the basis of our invention described herein. Thus, we envision that the non-mammalian GnRH (SEQ ID NO: 6, and SEQ ID NO: 7) and its analogs (SEQ ID NO: 2 and SEQ ID NO: 4) or biometics or interactions with its specific non-mammalian GnRH receptor when appropriately formulated and administered can be used to stimulate or inhibit immune function. A. Chen, Y. Ganor, S Rrahimipour, N. Ben-Aroya, Y. Koch, M; Levite, 2002 The neuropeptides GnRH-II and GnRH-I are produced by human T cells and trigger taminin receptor gene expression, adhesion, chemotaxis and homing to specific organs. Nature Medicine 8:1421-1426.

### SUMMARY OF THE INVENTION

The present invention is as set out in the claims.

The present disclosure, in a general and overall sense, relates to novel pharmaceutical preparations that include non-mammalian GnRH (SEQ ID NO: 6 and SEQ ID NO:7), its analogs (SEQ ID NO: 2 and SEQ ID NO: 4), long-acting formulations and biometrics, specifically designed to be useful in the immune system and in immune system disorders, such as relates to allergies and asthma, graft versus host disease, immune deficiency diseases, and autoimmune diseases, inflammation and tumor rejection, immune processes regulating implantation and pregnancy, endometrious, uterine fibroids, and immune involved disesase. These formulations are designed to be stable in blood or tissue and resistant to degradation by peptidases or other enzymes.

The non-mammalian GnRH (SEQ ID NO: 6 and SEQ ID NO: 7) or its analogs (SEQ ID NO: 2 and SEQ ID NO: 4) or biometics or long-acting formulations of non-mammalian GnRH (SEQ ID NO: 6 and SEQ ID NO: 7) of the present invention may act either as an agonist of non-mammalian GnRH with acute direct action on the immune system or as an antagonist using chronic delivery at immune system receptors leading to down regulation, or as a pure antagonist of immune system non-mammalian GnRH at the non-mammalian GnRH receptor.

The inventor has designed non-mammalian GnRH analogs (SEQ ID NO: 2) that are active in the immune system. The receptor binding activity of these particularly designed non-mammalian GnRH analogs (SEQ ID NO: 2) has also been characterized in the development of the present analogs. The analogs (SEQ ID NO: 2) of the invention may be further defined as resistant to enzymatic degradation by enzymatic activity of peptidases or other enzymes. The agonist and antagonists with the greatest receptor affinity and tissue and blood stability are expected to effectively regulate the immune system. The non-mammalian GNRH analogs (SEQ ID NO: 2) of the invention may be used to acutely stimulate or chronically inhibit over-activity of the immune system. The effects of the present non-mammalian GnRH analogs (SEQ ID NO:2 ) may thus be used to treat such immune disorders as allergies and asthma, graft versus host disease, immune deficiency diseases, and autoimmune diseases, inflammation, tumor rejections and immune processes regulating implantation and pregnancy.

In one aspect, the invention provides methods of designing analogs of non-mammalian GnRH (SEQ ID NO: 2) having increased activity in the immune system. Methods to acutely stimulate or chronically inhibit overactivity by the immune system are provided in another aspect of the present invention. The use of these analogs (SEQ ID NO: 2) directly on the immune system is yet another particular embodiment of the invention. The use of theme analogs (SEQ ID NO: 2) to directly affect immune system function is yet another embodiment of the invention. The use of these analogs (SEQ.ID NO. 2) to alter either or both the innate or adaptive immune system is yet another embodiment of the invention. The use of non-mammalian GnRH (SEQ ID NO: 6: ), its analogs (SEQ ID NO: 2), long-acting formulations and biometics to effect the function of tissues acting in the innate and adaptive immune system such as bone, lymph nodes, circulating leukocytes and lymphocytes, mast cells, natural killer cells, spleen T-cell, B-cell; and antibody production is yet another embodiment of the invention. The use of non-mammalian GnRH (SEQ ID NO: 6), its analogs (SEQ ID NO: 2), long-acting formulations and biometics to eftect the function of the cells of the innate and adaptive immune system including myeloid and lymphoid cells such as T-cell, B-cell, and antibody production is yet another embodiment of the invention.

It is also an embodiment of the present invention that the non-mammalian GnRH (SEQ ID NO: 6), its analogs (SEQ ID NO: 2), long-acting formulations and biometics of the present invention be used in pharmaceutical preparations to treat immune system disorders.

Non-mammalian GnRH (SEQ ID NO: 6), its analogs (SEQ ID NO: 2 ), long-acting formulations and biometics that are superagonists or antagonists at the immune system level constitute yet other embodiments of the invention. Such a non-mammalian GnRH (SEQ ID NO: 6), its analogs (SEQ ID NO: 2 ), long-acting formulations and biometics would provide for the inhibition of immune system overactivity, but could also stimulate if given acutely. The non-mammalian GnRH (SEQ ID NO: 6), its analogs (SEQ ID NO: 2), long-acting_{:} formulations and biometics, of the present invention thus comprise peptides that are capable of specifically binding the immune system GNRH) receptors with high affinity, are resistant to degradation by peptidases and effect either a down-regulation of the GnRH receptor or act as a true antagonist; inhibiting T-cell, B-cell or antibody production. The use of non-mammalian GnRH (SEQ ID NO: 6), its analogs (SEQ ID NO: 2), long-acting formulations and biometics to alter either or both the innate or adaptive immune system is yet another embodiment of the invention. The use of non-mamalian GnRH. (SEQ ID NO: 6), its analogs (SEQ ID NO: 2), long-acting. formulations and biometics to effect the function of T-cell, B-cell, and antibody production is yet another embodiment of the invention. The use of non-mammalian GnRH (SEQ ID NO: 6 ),its analogs (SEQ ID NO: 2), long-acting formulations and biometics to effect the function of T-cell,-B-cell, and antibody production is yet another embodiment of the invention.

In other embodiments, the invention comprises non-mammalian GnRH analogs, more specifically a GnRH II analog (SEQ ID NO: 2) that are modified at the C-terminal, which both show greater affinity for the immune system receptor than GnRH I (SEQ ID NO: 5). An ethylamide or aza-Gly¹⁰-NH₂ substitution may be used making the sequence more stable in the circulation and in the immune system and lymph. In other embodiments the non-mammalian GnRH analog sequence is substituted at the 6-position with a D-Arg or other D-amino acid. In yet other embodiments, both of these modifications are made to the non-mammalian GnRH) analog peptide sequence. The GnRH II (SEQ ID NO: 6) backbone and the substitutions of the molecule are expected to enhance the binding of the molecule, while at the same time the substitutions are designed to inhibit any of the peptidases that are present in lymph. These non-mammalian GnRH analogs (SEQ ID NO: 2) are expected to have increased binding to immune, system receptors. The immune system production of non-mammalian GnRH (SEQ ID NO: 6) and its receptor binding and the biological activity for the innate and adaptive immune system, including, but not limited to, the cells of the spleen, myeloid and lymphoid progenitors, such as monocytes, macrophages, and natural killer cells are expected to be increased. The biological activity of non-mammalian GnRH (SEQ ID NO: 6) and its analogs (SEQ ID NO: 2) on the innate and adaptive immune system's cells are being studied for each of these specially designed non-mammalian GnRH isoforms (SEQ ID NO: 6 ) and analogs (SEQ ID NO: 2) and compared to the closely related pituitary GnRH I analog (Buserilin)(SEQ ID NO: 10). These studies are expected to demonstrate greater stability of the non-mammalian, GnRH analogs (SEQ ID NO: 2), binding affinity and bioactivity compared to the GnRH I analogs examined.

In other embodiments, the invention provides non-mammalian GnRH (SEQ ID NO: 6 ), its analogs (SEQ ID NO: 2), long-acting formulations and biometics with enhanced activity within the tissues of the immune system and lymphatic system as well as the bone and spleen. This can include, but is not limited to, enhanced activity with T cells, monocytes, macrophages, dendritic, mast and natural killer cells.

In addition,a method for regulating T-cell, B-cell, and antibody production is provided with the present non-mammalian GnRH (SEQ ID NO: 6), its analogs (SEQ ID NO: 2),long-acting formulations and biometics. The activity of the non-mammalian GnRH (SEQ ID NO: 6), its analogs (SEQ ID NO: 2) , long-acting formulations and biometics may be useful in the management of immune system disorders. The non-mammalian GnRH (SEQ ID NO: 6), its analogs (SEQ ID NO: 2), long-acting formulations and biometics also have a direct action on immune system tissue. This activity may prove beneficial in treatments for immune system disorders.

It is envisioned that the non-mammalian GnRH (SEQ ID NO: 6), its analogs (SEQ ID NO: 2), long-acting formulations and biometics will be administered intravenously, intra-nasally, orally, transdermally, subcutaneously, vaginally or intramuscularly. However, virtually any mode of administration may be used in the practice of the invention.

Another embodiment of the invention provides non-mammalian-GnRH (SEQ ID NO: 6 ) its analogs (SEQ ID NO: 2), long-acting formulations and biometics that are resistant to degradation by peptidases. This isoform or analog will bind the immune system GnRH I (SEQ ID NO: 5) receptor or non-mammalian GnRH (SEQ ID NO: 6 and SEQ ID NO: 7) receptor with high affinity so to first stimulate then down-regulate the receptor to displace the endogenous GnRH-tike activity and block its action.

In another aspect, the invention provides more potent non-mammalian GnRH (SEQ ID NO: 6), its analog (SEQ ID NO: 2) long-acting formulations and biometics that will specifically bind to the immune system GnRH receptor. In addition, these-formulations are stable in the lymphatic circulation. The existing mammalian GnRH analogs are proline-containing molecules. Since human pituitary, blood and lymph contain an enzymatic activity that similarly degrades GnRH at the 5-6 position, not at the 9-10 position, the present non-mammalian GnRH analogs (SEQ ID NO: 2) have been designed to inhibit the former enzymatic activities, and have substitutions in the 5-6 position of the molecule. Some of the analogs also have a substitution at the 10 position with an ethylamide which is only a weak inhibitor of the post-proline peptidase. Aza-Gly¹⁰-NH₂, inhibits degradation by post-proline peptidase. Zohar Y, Goren A, Fridkin M, Ethahati E, Koch Y 1990 Degradation of gonadotropin-releasing hormones in the gilthead seabream, Sparus aurata. 11. Cleavage of native salmon GnRH, mammalian LHRH, and their analogs in the pituitary, kidney, and liver. Gen Comp Endocrinol 79:306-319; Benuck M, Marka N 1976 Differences in the degradation of hypothalamic, releasing factors by rat and human serum. Life Sci 19:1271-1276.

The stability of the present non-mammalian isoforms or analogs in the presence of peptidases and immune system tissues was also examined. The direct measurement of non-mammalian GnRH (SEQ ID NO: 6 and SEQ ID NO: 7) and its analogs (SEQ ID NO: 2 and SEQ ID NO: 4) were examined. Replacement of Gly¹⁰-NH₂ with aza-Gly-NH₂ made each of these GnRH analogs more resistant to degradation. It was found that the less active ah analog is as a competitor for GnRH degradation by peptidase, the more stable that analog will be in the immune system tissues and in lymph. Thus, the existing GnRH I analogs commonly used in medicine can be degraded much more rapidly in the immune system and lymph.

The findings of inhibition of overactivity by the immune system can be explained by recognizing that the decapeptide sequence for GnRH I (SEQ ID NO: 5) is not the only active GnRH sequence in the immune system. Substantial data exist that there is a GnRH receptor for which GnRH I (SEQ ID NO: 5) has low affinity, and that there is a GNRH) of which the chemical nature is not identical to GnRH I (SEQ ID NO: 5). It is postulated that a different immune system GnRH from the GnRH I (SEQ ID NO: 5) exists and that there is one or more immune system receptors that prefers this immune system GnRH. GnRH I (SEQ ID NO: 5) acts as a partial agonist of immune system GnRH. When receptors are available, it acts as an agonist of immune system GnRH. When immune system receptors are low or occupied, GnRH I (SEQ ID NO: 5) competes with the more potent immune system GnRH resulting in an antagonistic action.

The present inventor has found that certain non-mammalian GnRH isoforms (SEQ ID NO: 6 and SEQ ID NO: 7) and analogs (SEQ ID NO: 2 and SEQ ID NO: 4) can act on the immune system GnRH receptor, and with high affinity binding, affect changes in the immune system environment that effect the immune system. This finding is the basis of the invention disclosed herein. Thus, the present Applicant has developed particular (non-mammalian) GnRH analogs (SEQ ID NO: 2 and SEQ ID NO: 4) that can be used to regulate the immune system.

In additional embodiments, the specificity, activity and stability of these analogs (SEQ ID NO: 2 and SEQ ID NO: 4) were investigated at the immune system. A production by and a direct action on immune system tissue were found. A potential direct action of these analogs (SEQ ID NO: 2 and SEQ ID NO: 4) is indicated. Such analogs (SEQ ID NO: 2 and SEQ ID NO: 4) could be used to treat immune system disorders.

It is envisioned that non-mammalian GnRH isoforms (SEQ ID NO: 6 and SEQ ID NO: 7) are produced in cells of the immune system and that certain cell types of the immune system have specific receptors that bind non-mammalian GnRH isoforms (SEQ ID NO: 6 and SEQ ID NO: 7) to regulate the function of the immune system both the innate and adaptive systems. We will be able to regulate the function of the immune systems by regulating the non-mammalian GnRH concentrations to which the immune system is exposed or by regulating the number and activity of the receptor to which the non-mammalian GnRH interacts.
One can regulate the concentrations of the non-mammalian GnRH by using agonists or antagonists, such as but not limited to, peptides or antibodies. Any functional mimetics may be used for any purpose as the non-mammalian GnRH analogs (SEQ ID NO: 2) of the present invention which can include, among other things, antagonizing the activity of GnRH receptor or as an antigen in a manner described elsewhere herein. Functional mimetics of the non-mammalian GnRH analogs (SEQ ID NO: 2) the present invention include but are not limited to truncated polypeptides or synthetic organic or inorganic molecules comprising a comparable. GnRH receptor binding site. Polynucleotides encoding each of these functional mimetics may be used as expression cassettes to express each mimetic polypeptide. It is preferred that these cassettes comprise 5' and 3' restriction sites to allow for a convenient means to ligate the cassettes together when desired. It is further preferred that these cassettes comprise gene expression signals known in the art or described elsewhere herein.

We also envision that one can regulate the concentration of the non-mammalian GnRH by blocking and/or stimulating it's expression, processing or release. Similarly, the activity of non-mammalian GnRH at the GnRH receptor may be blocked or enhanced by altering the receptor or its expression at the molecular level, to include but is not limited to the DNA or RNA expression. One may also block the receptor translation, processing or assembly to reduce its activity or number, or to stimulate its activity or number.

The ligand or receptor activity, expression, translation, or processing may be adjusted to affect the process of a disease which is the result of immune system function or dysfunction. The detection of or the monitoring of a non-mammalian GnRH (SEQ ID NO: 6 and SEQ ID NO: 7) or its receptor of use to follow a process be it physiological or pathophysiological or to detect a pathophysiology resulting from or effecting a disorder or function of non-mammalian GnRH or the receptor in the immune system.

### BRIEF DESCRIPTION OF DRAWINGS

Fig. 1 shows localization of GnRH II in the human spleen.
Fig. 2 is a graph showing stability of GnRH II analog in blood and plasma. GnRH II Analog is stable throughout twenty-four hour of incubation in plasma and for the seventeen hours in serum following the initial serum formation.
Fig. 3 is a graph showing antibody response for GnRH II analog and normal response titre and II-6 response. The IL-6 response in rabbit serum is shown following booster immunization with GnRH II analog (|—|), or C-ase-1 (Δ---Δ)
Fig. 4 is a graph showing the effect of GnRH isoforms on leukocyte function. The effect of GnRH II and I on GM-CSF release from human leukocytes at 3 and 20 hours is compared.
Fig. 5 is a graph showing the effect of GnRH II analog on leukocyte function. The effect of GnRH II analog and Buserelin on GM-CSF release from human leukocytes at 3 and 20 hours is compared.
Fig. 6 shows localization of GnRH II receptor in immune tissues.

### DETAILED DESCRIPTION OF THE PREFERRED EMBODIMENTS

Following long-standing patent law convention, the terms "a" and "an" mean "one or more" when used in this application, including the claims.

For purposes of describing the present invention the "immune system" includes several specialized cells which rid the body of infection. The "immune system" includes several lymphoid organs and tissues throughout the body such as the spleen, bone marrow, thymus, tonsils, adenoids, appendix, Peyers patches, lymphatic vessels and lymph nodes which carry white blood cell containing lymph. The lymphatic vessels collect and move lymph into the blood circulation. The spleen and lymph nodes provide a collection point for immune system cells that fight infection. Lymphoid tissue is contained throughout the body in the bone marrow, thymus, tonsils, adenoids, Peyer's patches, and appendix.

The "immune system" can be divided into the "innate system" and the "adaptive system". The cellular components of both systems originate from the same progenitor, hematopoietic stem cells, in the bone marrow and differentiates to the myeloid or lymphoid progenitors cells. The myeloid progenitor cells give rise to precursors of granulocytes (neutrophils, basophils, eosinphils), monocyctes, immature dendritic and other poor described precursors found in blood. The granulocytes are stimulated during infection and inflammation and leave the blood to the site for innate immune response, including phagocytosis, allergic inflammation. The monocyctes, dendritic and other poorly described precursors enter tissues and also play an important role in the innate immune system. The monocytes further differentiate to macrophages upon entry into tissues and through phagocytosis play a critical role in the innate immune system. The dendritic cells also take up antigen and deliver it to the lymphocytes. The dendritic cells regulate the function of the natural killer cells. Mast cells also differentiate in tissues near small vessels and release substances that regulate vascular permeability (allergic responses). Thus, the innate immune system is the first response to infection, inflammation and foreign antigens.

The lymphoid progenitor cells give rise to B cells (differentiate in the bone) and T cells (differentiate in the thymus) and natural killer cells. The B cells can be stimulated to produce antibodies and T cells further differentiate on activation into cytotoxic (killer) T cells that kill infected cells or helper Tcells that activate B cells and macrophages. The natural killer cells are of the lymphoid progenitor lineage, but lack antigen-specificity. These lymphocytes circulate in the blood and peripheral lymphoid tissues and make up the adaptive immune system, i.e. the memory response and humoral response to infection and foreign antigens.

The lymphoid organs, the spleen, nodes, mucosal, tonsils, adenoids, appendix, Peyer's patches assist in the adaptive immune system by trapping antigens that are carried for the peripheral tissues by activity of the innate immune system's macrophages and dendritic cells, which present the antigen to the lymphocytes.

The oldest system on an evolutionary scale is the innate system. GnRH II (SEQ ID NO: 6) is believed to be primarily involved in this system. The adaptive system is a newer developed system and it is believed that both GnRH I (SEQ ID NO: 5) and II (SEQ ID NO: 6) receptors may exist for this system.

Lymphocytes, within the immune system are produced in the bone marrow. The two types of lymphocytes include B-cells and T-cells. These cells are named for their place of maturity. The B-cells remain in the bone marrow until maturity while the T-cells mature in the thymus. The B-cells are part of the humoral immunity (antibody mediated immunity) and produce antibodies that circulate throughout the circulatory and lymphatic systems and attach to antigens (foreign matter) labeling them for attack by other immune system cells. The antibodies produced by the B-cells have specialized regions that can target and bind to various antigens. Once the antibodies bind to the antigens the antigen-antibody complex activates the complement protein cascade which involves nine complement proteins. The complement proteins assist antibodies in removing the antigen from the body.

The T-cells vary in function depending on their type. There are three types of T-cells-helper T-cells, killer T-cells and suppressor T-cells. Helper T-cells (CD4+ T-cells) are necessary to activate the B-cells for the production of antibodies. They can also activate other T-cells and immune system scavenger cells such as macrophages and influence which type of antibody is produced. Killer T-cells (CD8+ T-cells or cytotoxic T-cells) attack and destroy diseased cells. These T-cells are responsible for cell-mediated immunity. Suppressor T cells help to regulate other processes of the immune system. T-cells depend on the major histocompatibility complex (MHC), unique cell surface molecules, to recognize antigen fragments.

After a macrophage engulfs and processes an antigen, the macrophage displays the antigen fragments combined with Class II MHC protein and Class I MHC on its cell surface. The antigen-Class II protein combination attracts a helper T-cell and promotes the helper T-cell's activation. A killer T-cell is attracted to and binds the antigen -Class I protein complex for activation.

A B-cell can bind to an antigen for which it has a compatible receptor. Once bound the B-cell will engulf the antigen and process it. The B-cell will then display a piece of the antigen bound to a Class II MHC protein on its cell surface. This complex can then bind to a helper T-cell. Once the complex binds the helper T-cell, the B-cell is converted into an antibody secreting plasma cell.

The term "acute inflammation" refers to an immediate and early response to an injurious agent which has a short duration and lasts from hours to days. It is characterized by alteration in the vascular caliber which leads to an increase in blood flow, changes in the microvasculature (capillaries) that allows plasma proteins and white blood cells to cross the capillary membrane to the interstitial space (vascular permeability), and aggregation of white blood cells in the focus of the injury (chemotaxis). This can be caused by bacterial infection, heat, cold, trauma, electricity, impact and chemicals.

The term "complement system" refers to a factor in inflammation that contains nine different proteins called "complement proteins" C1-C9. The complement system can be activated by one of two ways being the classical pathway and the alternate pathway. The classical pathway is triggered by the interaction of an antibody with an antigen. The C1 macromolecule is a Ca⁺⁺-dependent complex of one Clq, two Clr, and two Cls molecules. The Cl macromolecule remains intact only when Ca⁺⁺ is present; otherwise the individual subunits dissociate from each other. Activation occurs when two of Clq's six monomers bind to the Fc regions of two IgG molecules or to one pentameric IgM molecule. Two IgG molecules must be properly spaced to cause activation, whereas a single pentameric IgM has that proximity built into its structure. Therefore, IgM is much more efficient at activating complement than IgG. Activity of IgG is in the order IgG₃ > IgG₁ > IgG₂. IgG₄ does not fix complement. Once Ig is bound to C1q, the C1q molecule undergoes change in tertiary structure, causing autocatalytic activation of C1r to C1r. C1r then cleaves a bond in C1s to produce C1s. No cleavage fragment is released when either Clr or C1s is cleaved. C1s is also called C1 esterase. C1s may cleave C4 into C4a and C4b. C4b, the major cleavage fragment, binds to membrane if membrane is present. C1s can then either cleave free C2 to produce C2a and C2b, which is an inefficient process, or cleave C2 in a C4b, C2 complex to produce C4b,C2a and free C2b, which is a very efficient process. C2a is the major cleavage fragment of C2. If free C2 has been cleaved, then C2a must bind to C4b to form a C4b,2a complex, or the C2a will decay and be inactive. C4b,2a is the classical pathway C3 convertase, which may cleave C3 into C3a and C3b. C2a contains the enzymatic site for cleavage of C3. C4b,2a requires the presence of magnesium and decays over time at physiologic temperatures.

The classical pathway can also be activated by mechanisms independent of Ab. Heparin (a polyanionic anticoagulant) and protamine (a polycation that is used to block heparin), when present in equimolar concentrations, can activate the classical pathway. Various other polyanions (eg, DNA and RNA) are thought to be able to react directly with C1q to activate the classical pathway. C-reactive protein is capable of leading to classical pathway activation without the presence of Abs. C1 bypass pathways have also been described, which do not use components of the classical pathway but result in C3 cleavage. One of these has been characterized as the MBL pathway.

The classical pathway is regulated by C1 esterase inhibitor (C1INH), which binds stoichiometrically (1:1) to C1r and C1s and to C1r and C1s to inactivate these proteins permanently. C1INH also binds stoichiometrically to plasmin, kallikrein, activated Hageman factor, and coagulation factor XIa. Its absence leads to hereditary angioedema. Factor J is a cationic glycoprotein that also inhibits C1 activity. C4-binding protein (C4BP) disassembles the C4b,2a complex, allowing factor I to inactivate C4b.

C3 convertase (eg, C3b,Bb) can become C5 convertase (eg, C3b,Bb,3b) by the addition of a C3b into the complex. C5 convertase cleaves C5 into C5a and C5b, beginning the formation of the membrane attack complex (MAC). C6 may then bind to C5b to produce C5b,6. Next, C7 may bind to form C5b,6,7, which can attach itself to membranes and lipid bilayers. When this occurs on a cell that does not otherwise have any complement products on it, this is called the innocent bystander phenomenon (and may cause hemolysis of the innocent cell). C8 may then bind to the C5b,6,7 complex to form C5b,6,7,8, which can cause slow, inefficient lysis of the cell. Finally, C9 binds to the complex to produce C5b,6,7,8,9, which initiates substantial lysis of the cell. As additional C9 molecules are added to the C5b-9 complex, lysis increases. The MAC is regulated by S protein, also called vitronectin (which controls the activity of C5b-7), by homologous restriction factor (HRF), by SP40,40, and by CD59 (which regulates C8,9 activity). C5a is a very powerful chemotactic factor which increases the vascular permeability of capillaries, C3a increases vascular permeability and has some chemotactic properties. C5a in conjunction with C3a cause release of histamine from mast cells which leads to vasodilation leading to redness in the area of injury.

The alternate pathway is activated by fungal products (from infections), some polysaccharides, and aggregated antibody molecules. It is essentially the same as the classical pathway, but does not require the antigen-antibody complex to activate it. The alternative pathway leads to the cleavage of C3, but depends on the constant cleavage of small amounts of C3 into C3a and C3b. C3b then serves as a substrate for factor B to produce the complex C3b,B. Factor D (an activated enzyme in plasma) cleaves factor B to produce C3b,Bb. Properdin (P) stabilizes this C3b,Bb complex to retard its decay.

The term "kinin system" refers to a system responsible for the formation of blood clots. Beginning with tissue injury, the Hageman Factor (Factor XIIa) is activated. This Factor XIIa (a) functions in the intrinsic clotting system, (b) converts plasminogen to plasmin (protease), and (c) converts prekallikrein to kallikrein (proteolytic enzyme). Once kallikrein is formed it converts high molecular weight kininogen to bradykinin. Bradykinin is responsible for arteriolar dilation, increased vascular permeability and pain.

The term "arachidonic acid" system refers to a system that begins when cell membrane phospholipids are broken down by phospholipases into arachidonic acid. Arachidonic acid can then be converted to prostaglandin G2 in the presence of cyclooxygenase and 5-hydroperoxyeicosatetraenoic acid (5-HPETE) in the presence of 5-lipoxygenase. Prostaglandin G2 (PGG2) is then converted to PGH2 in the presence of PGH synthase. PGH2 then converts to PGD2 (vasodilator), PGE2 (vasodilator causes pain), PGF2α (vasodilator) and thromboxane (vasoconstrictor) in the presence of enzymes PGH-PGD isomerase, PGH-PGE isomerase, reductase, TxA synthase and prostacyclin synthase respectively. 5-HPETE can be converted to leukotriene A4 (LTA4) in the presence of LTA4synthase. LTA4 is then converted to leukotriene B4 (LTB4) in the presence of LTA4 hydrolase and LTC4 in the presence of LTC4 synthase. LTC4 is then converted to LTD4 in the presence of gamma-glutamyl transferase. LTD4 is then converted to LTE4 in the presence of peptidase. LTC4 and LTD₄ are potent contracting agents of smooth muscle in airways and blood vessels; in addition, they induce mucus secretion and promote plasmatic exudation with direct action on endothelial cells. LTB₄ is a potent chemokinetic and chemotactic agent.

The term "factors in inflammation" refers to the complement system, the kinin system and the arachidonic acid system.

The term "white blood cells" refers to granulocytes and agranulocytes. The granulocytes include neutrophils, eosinophils, and basophils with the neutrophils being the only phagocytic granulocyte. The agranulocytes include lymphocytes and monocytes (macrophages when in the interstitial space) with the macrophages being the only phagocytic agranulocyte.

The term "opsonization" refers to the process of bacteria being coated with antibody (IgG) or C3b which then greatly facilitates phagocytosis.

The term "Mendelian or genetic disorders" refers to single gene defect disorders which can encompass any of four possible types. These types include autosomal dominant, autosomal recessive, X-linked dominant, and X-linked recessive. For autosomal dominant (AD) one copy of defective gene is all that is needed for symptoms. For autosomal recessive (AR) two copies of defective gene is needed for symptoms. For X-linked dominant (XD) one copy of defective gene on sex chromosome is needed for symptoms. For X-linked recessive (XR) two copies of defective gene on sex chromosome is needed for symptoms.

An "immune system disorder" includes allergies and asthma, graft versus host disease, immune deficiency diseases, and autoimmune diseases.

"Allergies", of which asthma is included, are brought about by production of high levels of the antibody, IgE. The IgE antibodies bind to two types of cells basophils and mast cells. These cells found in the lungs, skin, tongue, and nose and intestinal tract are then stimulated to release histamines that cause allergy symptoms.

"Graft versus host disease" is seen in tissue transplants. When tissues or organs are transplanted from one person to another, the recipient's immune system attempts to rid itself of the foreign matter in a process termed "rejection". In addition, the transplanted immune cells can attack the tissues of the recipient which is called graft versus host disease. There are a group of HLA antigens on each person's tissue and organs which are specific to that given person. This group of HLA antigens is used in tissue typing to determine compatibility for organ and tissue transplants.

"Immune deficiency diseases" result when one or more parts of the immune system are missing. These diseases can be acquired in many different ways such through inheritance, through infections or other illnesses, or as a side effect of drug treatment. An immunodeficiency can be either primary or secondary. Primary immunodeficiency is classified into four main groups depending on which component of the immune system is deficient: B cells, T cells, phagocytic cells, or complement. Considerable heterogeneity may exist within each disorder. T-cell defects include several disorders with associated B-cell (antibody) defects, which is understandable since B and T cells originate from a common primitive stem cell and T cells influence B-cell function. Phagocytic diseases include disorders in which the primary defect is one of cell movement (chemotaxis) and those in which the primary defect is one of microbicidal activity.

"B-cell deficiencies" include X-linked agammaglobulinemia, Ig deficiency with hyper IgM (XL), IgA deficiency, IgG subclass deficiencies, antibody deficiency with normal or elevated Igs, immunodeficiency with thymoma, common variable immunodeficiency, transient hypogammaglobulinema of infancy. "Predominant T-cell deficiencies" include DiGeorge anomaly, chronic mucocutaneous candidiasis, Nezelof syndrome, nucleoside phosphorylase deficiency (AR), natural killer cell deficiency, idiopathic CD4 lymphocytopenia. "Combined B-cell and T-cell diseases" include severe combined immunodeficiency(AR or XL), adenosine deaminase deficiency (AR), reticular dysgenesis, bare lymphocyte syndrome, ataxia-telangiectasia (AR), WIskott-Alrich syndrome (XL), short-limbed dwarfism. "Phagocytic disorders" include defects of cell movement and defects of microbicidal activity. Defects of cell movement include hyperimmunoglobulinema E syndrome, leukocyte adhesion defect type 1 (AR). Defects of microbicidal activity include chronic granulomatous disease (XL or AR), neutrophil G6PD deficiency, myeloperoxidase deficiency (AR), Chediak-Higashi syndrome (AR). "Complement disorders" include defects of complement components and defects of control proteins. Defects of complement components include C1q, C1rs, C1s, C2, C3, C4, C5, C6, C7, C8 and C9 deficiency (ACD). Defects of the control proteins include C1 inhibitor defiency (AD), factor 1 (C3b inactivator) defiency (ACD), factor H defiency (ACD), factor D deficiency (ACD), and properdin deficiency (XL).

"Secondary immunodeficiency" is an impairment of the immune system resulting from an illness in a previously normal person. The impairment often is reversible if the underlying condition or illness resolves. Secondary immunodeficiencies are considerably more common than primary immunodeficiencies and occur in many hospitalized patients. Nearly every prolonged serious illness interferes with the immune system to some degree. There are numerous predisposing factors to secondary immunodeficiencies. These include hereditary and metabolic diseases, immunosuppressive agents, infectious diseases, infiltrative and hematologic disease, surgery and trauma. The hereditary and metabolic diseases include chromosome abnormalities, uremia, diabetes mellitus, malnutrition, vitamin and mineral deficiencies, protein-losing enteropathies, nephrotic syndrome, myotonic dystrophy and sickle cell disease. Immunosuppressive agents that can cause secondary immunodeficiencies include radiation, immunosuppressive drugs, corticosteroids, anti-lymphocyte or anti-thrombocyte globulin, anti-T-cell monoclonal antibodies. Certain infectious diseases include congenital rubella, viral exanthems, HIV infection, cytomegalovirus infection, infectious mononucleosis, acute bacterial disease, severe mycobacterial or fungal disease. Infiltrative and hematological diseases include histiocytosis, sarcoidosis, Hodgkin's disease and lymphoma, leukemia, myeloma, agranulocytosis and aplastic anemia. Surgery and trauma that can cause secondary immunodeficiency diseases include burns, splenectomy, anesthesia. Some miscellaneous disorders include systemic lupus erythamotosis, chronic active hepatitis, alcoholic cirrhosis, aging, anticonvulsant drugs, and graft versus host disease.

"Autoimmune diseases" arise when the immune system mistakes self tissues for non-self tissues and attacks it. Autoimmune disorders include alopecia, ankylosing spondylitis, antiphospholipid syndrome, autoimmune Addison's disease, autoimmune hepatitis, Behcet's disease, bullous pemphigoid, cardiomyopathy, celiac sprue-dermatitis, chronic fatigue immune dysfunction syndrome, chronic inflammatory demyelinating polyneuropathy, churg-Strauss syndrome, cicatricial pemphigoid, CREST syndrome, cold agglutinin disease, Crohn's disease, discoid lupus, essential mixed cryoglobulinemia, fibromyalgia-fibromyositis, Grave's disease, Guillain-Bane, Hashimoto's thyroiditis, idiopathic pulmonary fibrosis, idiopathic thrombocytopenia purpura, IgA nephropathy, insulin dependent diabetes, juvenile arthritis, lichen planus, lupus, Meniere's disease, mixed connective tissue disease, multiple sclerosis, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, rheumatoid arthritis, sarcoidosis, scleroderma, Sjogren's syndrome, Stiff-Man syndrome, Takayasu arteritis, temporal arteritis/giant cell arteritis, ulcerative coligis, uveitis, vasculitis, vitiligo, and Wegener's granulomatosis.

An "isolated nucleic acid" is a nucleic acid the structure of which is not identical to that of any naturally occuring nucleic acid or to that of any fragment of a naturally occuring genomic nucleic acid spanning more than three separate genes. The term therefore covers, for example, (a) a DNA which has the sequence of part of a naturally occuring genomic DNA molecule, but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occuring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Specifically, excluded from this definition are nucleic acids present in mixtures of (i) DNA molecules, (ii) transfected cells, and (iii) cell clones, e.g., as these occur in a DNA library such as a cDNA or genomic DNA library.

The terms "complementary" or "complementarity" refer to the natural binding of polynucleotides by base pairing. For example, the sequence 5'-AGT-3' binds to the complementary sequence 3'-TCA-5'. Complementarity between two single-stranded molecules may be "partial" such that only some of the nucleic acids bind or it may be "complete" such that total complementarity exists between the single stranded molecules. The degree of complementarity between the nucleic acid strands has significant effects on the efficiency and strength of the hybridization between the nucleic acid strands.

The term "expression modulating fragment," EMF, means a series of nucleotides which modulates the expression of an operably linked ORF or another EMF. As used herein, a sequence is said to "modulate the expression of an operably linked sequence" when the expression of the sequence is altered by the presence of the EMF. EMFs include, but are not limited to, promoters, and promoter modulating sequences (inducible elements). One class of EMFs are nucleic acid fragments which induce the expression of an operably linked ORF in response to a specific regulatory factor or physiological event.

The terms "nucleotide sequence" or "nucleic acid" or "polynucleotide" or "oligonucleotide" are used interchangeably and refer to a heteropolymer of nucleotides or the sequence of these nucleotides. These phrases also refer to DNA or RNA of genomic or synthetic origin which may be single-stranded or double-stranded and may represent the sense or the antisense strand, to peptide nucleic acid (PNA) or to any DNA-like or RNA-like material. Generally, nucleic acid segments provided by this invention may be assembled from fragments of the genome and short oligonucleotide linkers, or from a series of oligonucleotides, or from individual nucleotides, to provide a synthetic nucleic acid which is capable of being expressed in a recombinant transcriptional unit comprising regulatory elements derived from a microbial or viral operon, or a eukaryotic gene. Probes may, for example, be used to determine whether specific mRNA molecules are present in a cell or tissue or to isolate similar nucleic acid sequences from chromosomal DNA as described by Walsh et al. (Walsh, P. S. et al., 1992, PCR Methods Appl 1:241-250). They may be labeled by nick translation, Klenow fill-in reaction, PCR, or other methods well known in the art. Probes of the present invention, their preparation and/or labeling are elaborated in Sambrook, J. et al., 1989, Molecular Cloning: A Laboratory Manual, Cold Spring Harbor Laboratory, NY; or Ausubel, F. M. et al., 1989, Current Protocols in Molecular Biology, John Wiley & Sons, New York N.Y., both of which are incorporated herein by reference in their entirety.

The term "open reading frame," ORF, means a series of nucleotide triplets coding for amino acids without any termination codons and is a sequence translatable into protein.

The terms "operably linked" or "operably associated" refer to functionally related nucleic acid sequences. For example, a promoter is operably associated or operably linked with a coding sequence if the promoter controls the transcription of the coding sequence. While operably linked nucleic acid sequences can be contiguous and in the same reading frame, certain genetic elements e.g. repressor genes are not contiguously linked to the coding sequence but still control transcription/translation of the coding sequence.

The term "translated protein coding portion" means a sequence which encodes for the full length protein which may include any leader sequence or any processing sequence.

The term "mature protein coding sequence" means a sequence which encodes a peptide or protein without a signal or leader sequence. The peptide may have been produced by processing in the cell which removes any leader/signal sequence. The peptide may be produced synthetically or the protein may have been produced using a polynucleotide only encoding for the mature protein coding sequence.

The term "derivative" refers to polypeptides chemically modified by such techniques as ubiquitination, labeling (e.g., with radionuclides or various enzymes), covalent polymer attachment such as pegylation (derivatization with polyethylene glycol) and insertion or substitution by chemical synthesis of amino acids such as ornithine, which do not normally occur in human proteins.

The term "variant"(or "analog") refers to any polypeptide differing from naturally occurring polypeptides by amino acid insertions, deletions, and substitutions, created using, e g., recombinant DNA techniques. Guidance in determining which amino acid residues may be replaced, added or deleted without abolishing activities of interest, may be found by comparing the sequence of the particular polypeptide with that of homologous peptides and minimizing the number of amino acid sequence changes made in regions of high homology (conserved regions) or by replacing amino acids with consensus sequence. Alternatively, recombinant variants encoding these same or similar polypeptides may be synthesized or selected by making use of the "redundancy" in the genetic code. Various codon substitutions, such as the silent changes which produce various restriction sites, may be introduced to optimize cloning into a plasmid or viral vector or expression in a particular prokaryotic or eukaryotic system. Mutations in the polynucleotide sequence may be reflected in the polypeptide or domains of other peptides added to the polypeptide to modify the properties of any part of the polypeptide, to change characteristics such as ligand-binding affinities, interchain affinities, or degradation/turnover rate.

Preferably, amino acid "substitutions" are the result of replacing one amino acid with another amino acid having similar structural and/or chemical properties, i.e., conservative amino acid replacements. "Conservative" amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. "Insertions" or "deletions" are preferably in the range of about 1 to 10 amino acids, more preferably 1 to 5 amino acids. The variation allowed may be experimentally determined by systematically making insertions, deletions, or substitutions of amino acids in a polypeptide molecule using recombinant DNA techniques and assaying the resulting recombinant variants for activity.

Alternatively, where alteration of function is desired, insertions, deletions or non-conservative alterations can be engineered to produce altered polypeptides. Such alterations can, for example, alter one or more of the biological functions or biochemical characteristics of the polypeptides of the invention. For example, such alterations may change the characteristics such as ligand-binding affinities, interchain affinities, or degradation/turnover rate. Further, such alterations can be selected so as to generate peptides that are better suited for expression, scale up and the like in the host cells chosen for expression. For example, cysteine residues can be deleted or substituted with another amino acid residue in order to eliminate disulfide bridges.

The terms "purified" or "substantially purified" as used herein denotes that the indicated nucleic acid or polypeptide is present in the substantial absence of other biological macromolecules, e.g., polynucleotides, proteins, and the like. In one embodiment, the polynucleotide or polypeptide is purified such that it constitutes at least 95% by weight, more preferably at least 99% by weight, of the indicated biological macromolecules present (but water, buffers, and other small molecules, especially molecules having a molecular weight of less than 1000 daltons, can be present).

The term "recombinant," when used herein to refer to a polypeptide or protein, means that a polypeptide or protein is derived from recombinant (e.g., microbial, insect, or mammalian) expression systems. "Microbial" refers to recombinant polypeptides or proteins made in bacterial or fungal (e.g., yeast) expression systems. As a product, "recombinant microbial" defines a polypeptide or protein essentially free of native endogenous substances and unaccompanied by associated native glycosylation. Polypeptides or proteins expressed in most bacterial cultures, e.g., E. coli, will be free of glycosylation modifications; polypeptides or proteins expressed in yeast will have a glycosylation pattern in general different from those expressed in mammalian cells.

The term "recombinant expression vehicle or vector" refers to a plasmid or phage or virus or vector, for expressing a polypeptide from a DNA (RNA) sequence. An expression vehicle can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an amino terminal methionine residue. This residue may or may not be subsequently cleaved from the expressed recombinant protein to provide a final product.

The term "recombinant expression system" means host cells which have stably integrated a recombinant transcriptional unit into chromosomal DNA or carry the recombinant transcriptional unit extrachromosomally. Recombinant expression systems as defined herein will express heterologous polypeptides or proteins upon induction of the regulatory elements linked to the DNA segment or synthetic gene to be expressed. This term also means host cells which have stably integrated a recombinant genetic element or elements having a regulatory role in gene expression, for example, promoters or enhancers. Recombinant expression systems as defined herein will express polypeptides or proteins endogenous to the cell upon induction of the regulatory elements linked to the endogenous DNA segment or gene to be expressed. The cells can be prokaryotic or eukaryotic.

The term "secreted" includes a protein that is transported across or through a membrane, including transport as a result of signal sequences in its amino acid sequence when it is expressed in a suitable host cell. "Secreted" proteins include without limitation proteins secreted wholly (e.g., soluble proteins) or partially (e.g., receptors) from the cell in which they are expressed. "Secreted" proteins also include without limitation proteins that are transported across the membrane of the endoplasmic reticulum. "Secreted" proteins are also intended to include proteins containing non-typical signal sequences (e.g. Interleukin-1 Beta, see Krasney, P. A. and Young, P. R. (1992) Cytokine 4(2):134-143) and factors released from damaged cells (e.g. Interleukin-1 Receptor Antagonist, see Arend, W. P. et. al. (1998) Annu. Rev. Immunol. 16:27-55)

Where desired, an expression vector may be designed to contain a "signal or leader sequence" which will direct the polypeptide through the membrane of a cell. Such a sequence may be naturally present on the polypeptides of the present invention or provided from heterologous protein sources by recombinant DNA techniques.

As used herein, "substantially equivalent" can refer both to nucleotide and amino acid sequences, for example a mutant sequence, that varies from a reference sequence by one or more substitutions, deletions, or additions, the net effect of which does not result in an adverse functional dissimilarity between the reference and subject sequences. Typically, such a substantially equivalent sequence varies from one of those listed herein by no more than about 35% (i.e., the number of individual residue substitutions, additions, and/or deletions in a substantially equivalent sequence, as compared to the corresponding reference sequence, divided by the total number of residues in the substantially equivalent sequence is about 0.35 or less). Such a sequence is said to have 65% sequence identity to the listed sequence. In one embodiment, a substantially equivalent, e.g., mutant, sequence of the invention varies from a listed sequence by no more than 30% (70% sequence identity); in a variation of this embodiment, by no more than 25% (75% sequence identity); and in a further variation of this embodiment, by no more than 20% (80% sequence identity) and in a further variation of this embodiment, by no more than 10% (90% sequence identity) and in a further variation of this embodiment, by no more that 5% (95% sequence identity). Substantially equivalent, e.g., mutant, amino acid sequences according to the invention preferably have at least.80% sequence identity with a listed amino acid sequence, more preferably at least 90% sequence identity. Substantially equivalent nucleotide sequences of the invention can have lower percent sequence identities, taking into account, for example, the redundancy or degeneracy of the genetic code. Preferably, nucleotide sequence has at least about 65% identity, more preferably at least about 75% identity, and most preferably at least about 95% identity. For the purposes of the present invention, sequences having substantially equivalent biological activity and substantially equivalent expression characteristics are considered substantially equivalent. For the purposes of determining equivalence, truncation of the mature sequence (e.g., via a mutation which creates a spurious stop codon) should be disregarded. Sequence identity may be determined, e.g., using the Jotun Hein method (Hein, J. (1990) Methods Enzymol. 183:626-645). Identity between sequences can also be determined by other methods known in the art, e.g. by varying hybridization conditions.

The term "antibody" includes whole antibodies and fragments thereof, single chain (recombinant) antibodies, "humanized" chimeric antibodies, and immunologically active fragments of antibodies (eg. Fab fragments).

The term"degenerate variant" means nucleotide fragments which differ from a nucleic acid fragment of the present invention (e.g., an ORF) by nucleotide sequence but, due to the degeneracy of the genetic code, encode an identical polypeptide sequence. Preferred nucleic acid fragments of the present invention are the ORFs that encode proteins. The amino acids and their corresponding DNA codons can include, but are not limited to, isoleucine (ATT, ATC, ATA), leucine (CTT, CTC, CTA, CTG, TTA, TTG), valine (GTT, GTC, GTA, GTG), phenylalahine (TTT, TTC), methionine (ATG), cysteine (TGT, TGC), alanine (GCT, GCC, GCA, GCG), glycine (GGT, GGC, GGA, GGG), proline (CCT, CCC, CCA, CCG), threonine (ACT, ACC, ACA, ACG), serine (TCT, TCC, TCA, TCG, AGT, AGC), tyrosine (TAT, TAC), tryptophan (TGG), glutamine (CAA, CAG), asparagine (AAT, AAC), histidine (CAT, CAC), glutamic acid (GAA, GAG), aspartic acid (GAT, GAC), lysine (AAA, AAG), and arginine (CGT, CGC, CGA, CGG, AGA, AGG)..

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE I - Design of Non-Mammalian GnRH Analogs

The present example outlines how analogs (SEQ ID NO: 2 and SEQ ID NO: 4) of non-mammalian GnRH with increased activity in immune system tissues are designed.

Existing GnRH I analogs are designed for activity at the pituitary GnRH receptor and with extended stability in the circulation of individuals. Yet, the existing data indicate that the immune system tissues have a high affinity GnRH receptor which differs from that in the pituitary. In addition, the degradation of GnRH I (SEQ ID NO: 5) is different in the immune system. Therefore, prior known pituitary GnRH I analogs have not been designed for use at immune system sites, and potent non-mammalian GnRH analogs have not been designed for use at immune system sites. The present invention provides potent non-mammalian GnRH analogs (SEQ ID NO: 2 and SEQ ID NO: 4) for use at immune systems sites.

Non-mammalian analogs of GnRH (SEQ ID NO: 2 and SEQ ID NO: 4) were synthesized by order. They were specifically designed to prevent degradation of the analog in immune system tissues. This allows for the maintenance of sufficient concentrations of analog to remain active when administered to the individual and to reach the immune system tissues. Due to the particular specificity of the immune system receptor and specific peptidase in blood and lymph, the particular analogs of the invention were designed. Analogs of the salmon GnRH (SEQ ID NO: 4) and GnRH II (SEQ ID NO: 2) sequences, that both show greater affinity for the immune system receptors than for the pituitary receptor, were modified to the tenth amino acid to ethylamide or aza-Gly¹⁰-NH₂ analog to make them resistant to degradation in the circulation and by peptidases. The GnRH II sequence (SEQ ID NO: 2) and the salmon GnRH sequence (SEQ ID NO: 4) were also modified at the 6 position using D-Arg, making them resistant to degradation by the peptidase in blood, and were modified at the 10 position making them stable in blood and the immune system tissues. These analogs (SEQ ID NO: 2 and SEQ ID NO: 4) are expected to have increased binding to the immune system receptors and increased metabolic stability.

### EXAMPLE II - Localization of Non-Mammalian GnRH in Tissues of the Immune System

Tissue of the immune system were examined for the presence of non-mammalian GnRH (SEQ ID NO: 6 and SEQ ID NO: 7) in their cells. The presence of non-mammalian GnRH (SEQ ID NO: 6 and SEQ ID NO: 7) in the tissues of the human other that the T cell has not been previously described. This presence demonstrated in immune cells of mammalian tissues that non-mammalian GnRH isoforms are produced in the mammals and that they are present in the immune system.

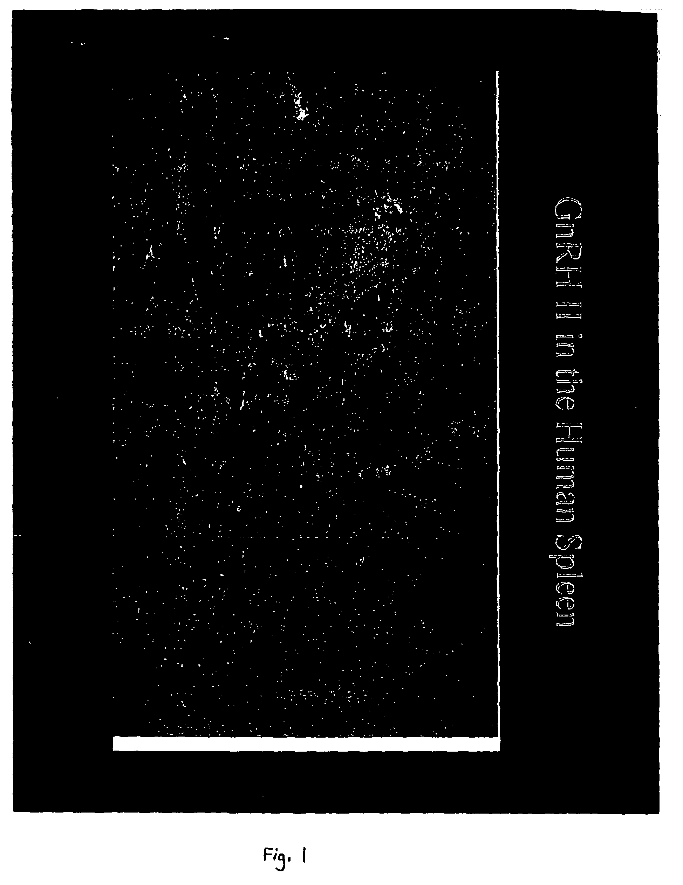

Human tissues from the thymus, spleen and lymph nodes were fixed and sectioned and plated by sections on glass slides. The human tissues on the glass slides were incubated with anti-GnRH II (1/100) for 1hour at RT. The tissues were then washed with phosphate buffered saline and anti-rabbit gamma globulin conjugated with biotin is incubated for 4 minutes at 55 C. The slide was rinsed in buffer followed by blocking of the endogenous peroxidase activity. Then streptavidin horse radish peroxidase was added and incubated for 4 minutes at 55 C. Stable diaminobenzidine (5 minutes at 55 C) was used to generate the signal. The slides were rinsed, mounted and read. The presence of GnRH II (SEQ ID NO: 6) was localized via the DAB using microscopy. In the immune tissues examined, spleen, thymus and lymph node GnRH II was visualized. See Fig. 1. Tissues such as atrium and liver were negative.

### Example III - Stability Studies of GnRH Analogs

The present example demonstrated the stability of the GnRH II analogs (SEQ ID NO: 2). The added stability of these non-mammalian analogs would effect a substantial increase in bioactivity.

The enzymatic degradation of the non-mammalian GnRH (SEQ ID NO: 6) and its analog (SEQ ID NO: 2) were studied using whole blood and plasma stability studies. A peptidase present in the immune system was used. Non-mammalian GnRH analogs (SEQ ID NO: 2) were designed with these specific criteria in mind. The stability of these non-mammalian GnRH analogs (SEQ ID NO: 2) to the enzymatic activity of the peptidase and in immune system cells were examined.

The stability of most potent receptor-active non-mammalian GnRH analogs (SEQ ID NO: 2) in the presence of peptidase and immune system cells was identified. Each of these analogs was then studied for their ability to resist degradation over time of incubation with the immune system cells at 37⁰ C. The reaction was stopped by freezing and the remaining GnRH I substrate, GnRH II substrate or non-mammalian analog was directly quantified by radioimmunoassay.

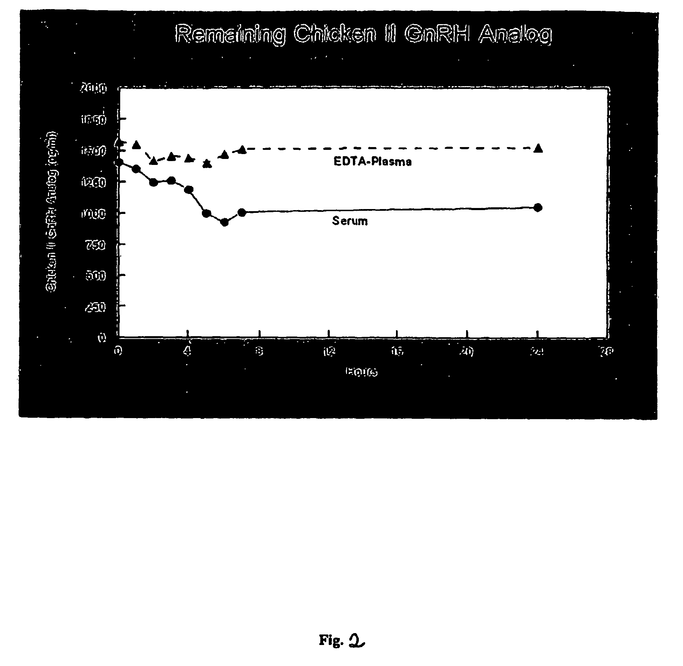

Studies using whole immune system cells were also performed. The enzymatic degradation of GnRH I (SEQ ID NO: 5) was studied as described above, replacing peptidase with immune system homogenate. Fig. 2 is a graph showing stability of GnRH II analog in blood and plasma.

### EXAMPLE IV - Inhibition of Antibody Response

The production of antibodies is a function of the immune system. The ability of the immune system to respond to substances perceived as foreign by the body with the production of specific antibodies which will effect the inactivation of the substance is a function of the immune system. Non-mammalian GnRH (SEQ ID NO: 6 and SEQ ID NO: 7) or its analogs (SEQ ID NO: 2 and SEQ ID NO: 4) can regulate this activity in a mammal and this is a novel activity. The chronic administration of non-mammalian GnRH activity can lead to the inhibition of the immune system's antibody response to a foreign substance.

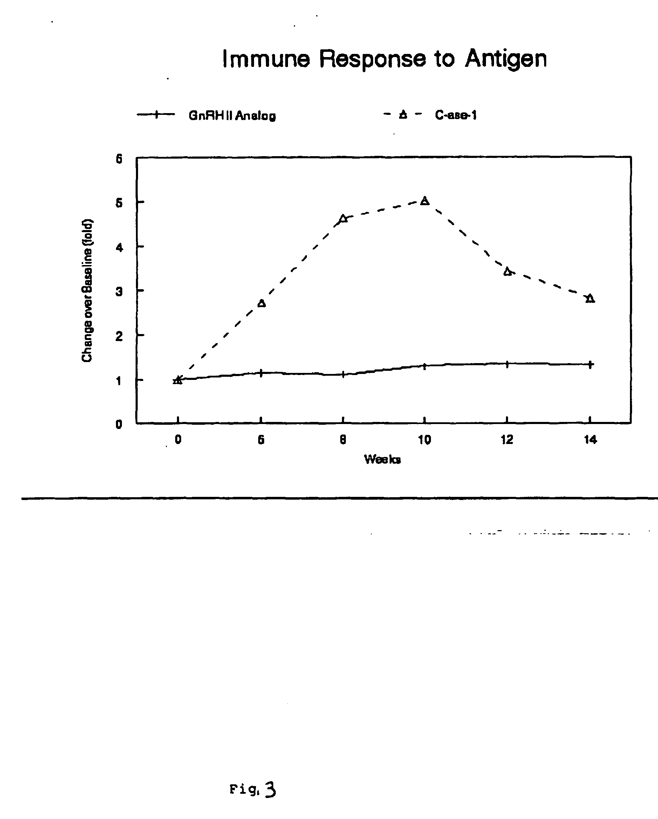

The very stable, long acting non-mammalian GnRH analog, D-Arg-GnRH II-aza-Gly-amide was conjugated to KLH and injected into rabbits to generate polyclonal antibodies. The titre of the antiserum was tested for binding to D-Arg-GnRH II-aza-Gly-amide and compared to the serum before treatment in each of four animals. This was compared to the generation of serum using proteins not conjugated to GnRH II (SEQ ID NO: 6). In three animals no antibodies were detected. In one of the four animals only an antibody of low titer was generated after four treatments, which inhibition occurred with continued immunization. Fig. 3 shows the antibody response for GnRH II analog (SEQ ID NO: 2) and normal response titre and Il-6 response.

### EXAMPLE V - Non-Mammalian GnRH and Methods for Treating Immune System Disorders

The present example defines a method by which the present invention may be used to treat immune system disorders of mammals. The mammal in some embodiments is a human. As a proposed dose regimen, it is anticipated that a human between 100 lbs and 150 lbs would be administered about 10 nanogram to 1.0 gram of GnRH II Analog (SEQ ID NO: 2) or salmon GnRH analog (SEQ ID NO: 4) or their natural isoforms with or without a release regulating carrier. This would be expected to be effective for treating immune system disorders in the mammal when administered.

We also envision the use of GnRH II (SEQ ID NO: 6) or its analogs (SEQ ID NO: 2) or biometics in a chronic fashion. It is anticipated that pulsatile administration will cause stimulation of its activity while chronic administration can be used to downregulate receptors leading to inhibition of non-mammalian GnRH activity.

In some embodiments, the dosing regimen will comprise a pulsatile administration of the GnRH II analog (SEQ ID NO: 2) over a 24-hour period, wherein the daily dosage is administered in relatively equal 1/24^{th} fractions. For example, where the daily dose is about 2.4 micrograms, the patient would be administered about 01 micrograms per hour over a 24-hour period. Such a daily pulsatile administration would create an environment in the patient sufficient to treat certain types of immune system disorders. The particular pharmaceutical preparations may be created by one of skill in the pharmaceutical arts, as described in Remington's Pharmaceutical Sciences Remington: The Science and Practice of Pharmacy, 19th edition, Vol. 102, A.R Gennaro, ed., Mack Publishing Co. Easton, PA (1995).

For purposes of practicing the present invention as an oligonucleotide in molecular biology applications, the non-mammalian GnRH analogs GnRH II (SEQ ID NO: 1) and salmon decapeptide GnRH analog cDNA sequences (SEQ ID NO: 3) would be employed, as described in the textbook of Sambrook, et al (1989) Molecular Cloning, A Laboratory Manual, 2d Ed., Cold Springs Harbor Laboratory, Cold Springs Harbor, N.Y. By way of example, the cDNA sequence for the non-mammalian GnRH of SEQ ID NO: 1 (GnRN II) or SEQ ID NO:3, (salmon GnRH) may be prepared as part of suitable vector, such as in an adenovirus or retroviral vector, and administered to the animal. Once the sequence is incorporated into the cell, the peptide product will be translated and peptide supplied. Because this method of treatment would not require that the peptide travel in the blood or lymph circulation in order to reach the site of action, there would be no requirement that the analog possess enzyme degradation resistance. This mode of treatment has not thus far been proposed, and hence the use of such a method in the treatment of immune system disorders is a novel clinical regimen.

### EXAMPLE VI - Use of Antibodies Specific for Non-Mammalian GnRH for Immune System Disorders

The present example demonstrates the utility for using the present invention non-mammalian GnRH decapeptides to prepare antibodies that preferentially bind the non-mammalian GnRH peptide sequences, or that bind the immune system non-mammalian GnRH peptide or protein, or the receptors therefor. It is also anticipated that these non-mammalian GnRH antibodies may be used in a variety of screening assays. For example, these antibodies may be used to determine levels of GnRH II in a sample as an indicator molecule. The levels of such non-mammalian GnRH (SEQ ID NO: 6 and SEQ ID NO: 7) may be used to monitor and follow a patient's immune system treatment. The antibodies to non-mammalian GnRH may be monoclonal or polyclonal antibodies.

These antibodies may be used for treatments that regulate the immune system via inhibiting the activity of non-mammalian GnRH. Polyclonal antibodies may be created by standard immunization techniques, wherein the immunogen used will be the GnRH II (SEQ ID NO: 6) or the salmon GnRH (SEQ ID NO: 7) decapeptide described herein. These peptides may be used either alone or together in a pharmaceutically acceptable adjuvant. The animal, such as a rabbit, would be administered several doses of the decapeptide preparation, and the levels of the animal=s antibody blood levels monitored until an acceptable antibody level (titer) had been reached.

For the preparation of monoclonal antibodies, one would follow standard techniques for the immunization of an animal, again using the peptides specific for non-mammalian GnRH. Once sufficiently high acceptable antibodies are reached (titer) in the animal, the spleen of the animal would be harvested, and then fused with an immortalized cell line, such as a cancer cell line, to produce a population of hybridoma cells. This hybridoma population of cells would then be screened for those that produce the highest amount of antibody that specifically bind the non-mammalian GnRH. Such hybridoma cells would be selected, and then cultured. The antibody to non-mammalian GnRH would then be collected from the media of the cell culture using techniques well know to those of skill in the art.

For purposes of the practice of preparing polyclonal and monoclonal antibody see the textbook Sambrook et al (1989) Molecular Cloning, A Laboratory Manual, 2nd Ed., Cold Springs Harbor Laboratory, Cold Springs Harbor, N.Y. All of the compositions and methods disclosed and claimed herein can be made and executed without undue experimentation in light of the present disclosure.

### EXAMPLE VII- Non-Mammalian GnRH Analogs or Biometics and Methods of Use in Treatment of Conditions of the Immune System

Due to the stability of the non-mammalian GnRH analogs, particularly GnRH II analog (SEQ ID NO: 2) and salmon GnRH analog (SEQ ID NO: 4), in the blood and lymph,the presence of binding receptors in immune system tissues, and their biological activity in immune system tissues, such analogs or biometics can be used in the treatment of conditions of or regulation of the immune system and the tissues therein. Such treatment or regulation may be for allergies or asthma, graft-versus-host disease, immunodeficiency disorders, and autoimmune disorders.

Conventional methods, known to those of ordinary skill in the art of medicine, can be used to administer the pharmaceutical formulation(s) to the patient. Typically, the pharmaceutical formulation will be administered to the patient by intramuscular injection, subdermal pellet, or nasal spray. The pharmaceutical formulation(s) can also be administered via other conventional routes (e.g., oral, subcutaneous, intrapulmonary, transmucosal, intraperitoneal, sublingual, or intrathecal routes) by using standard methods. In addition, the pharmaceutical formulations can be administered to the patient via injection depot routes of administration such as by using 1-, 3-, or 6-month depot injectable or biodegradable materials and methods.

Regardless of the route of administration, the therapeutical agent typically is administered at a daily dosage of 0.001 µg to 30 mg/kg of body weight of the patient. The pharmaceutical formulation can be administered in multiple doses per day, if desired, to achieve the total desired daily dose or as a long acting depot. The effectiveness of the method of treatment can be assessed by monitoring the patient for known signs or symptoms of the disorder.

### EXAMPLE VIII - Use of Antibodies Specific for Non-Mammalian GnRH Receptor for Immune System Disorders

The antibodies specific for non-mammalian GnRH receptor can be used to regulate immune system function. The present example demonstrates the utility for using the present invention non-mammalian GnRH receptor to prepare antibodies that preferentially bind the GnRH receptor peptide sequences, or that bind the immune system GnRH receptor peptide or protein. It is anticipated that these non-mammalian GnRH receptor antibodies may be used in a variety of screening assays. For example, these antibodies may be used to determine levels of GnRH II (SEQ ID NO: 6), or the GnRH receptor that binds GnRH II, in a sample as an indicator molecule. The levels of such GnRH may be used to monitor and follow a patient's immune system treatment. The antibodies to non-mammalian GnRH may be monoclonal or polyclonal antibodies.

These antibodies may be used for treatments that regulate the immune system via inhibiting the non-mammalian GnRH or the activity of the non-mammalian GnRH receptor. Other antiserum may interact with the non-mammalian GnRH receptor to stimulate it activity. Polyclonal antibodies may be created by standard immunization techniques, wherein the immunogen used will be peptides specific to the non-mammalian GnRH receptor. These peptides may be used either alone or together in a pharmaceutically acceptable adjuvant. The animal, such as a rabbit, would be administered several doses of the peptide preparation, and the levels of the animal's antibody blood levels monitored until an acceptable antibody level (titer) had been reached.

For the preparation of monoclonal antibodies, one would follow standard techniques for the immunization of an animal, again using peptides specific to the non-mammalian GnRH receptor. Once sufficiently high acceptable antibodies are reached (titer) in the animal, the spleen of the animal would be harvested, and then fused with an immortalized cell line, such as a cancer cell line, to produce a population of hybridoma cells. This hybridoma population of cells would then be screened for those that produce the highest amount of antibody that specifically bind the non-mammalian GnRH receptor. Such hybridoma cells would be selected, and then cultured. The antibody to non-mammalian GnRH receptor would then be collected from the media of the cell culture using techniques well know to those of skill in the art.

For purposes of the practice of preparing polyclonal and monoclonal antibody, see the textbook Sambrook et al (1989) Molecular Cloning, A Laboratory Manual, 2nd Ed., Cold Springs Harbor Laboratory, Cold Springs Harbor, N.Y. All of the compositions and methods disclosed and claimed herein can be made and executed without undue experimentation in light of the present disclosure.

### EXAMPLE IX Identification of the Non-Mammalian GnRH Receptor in Human Immune System Tissues

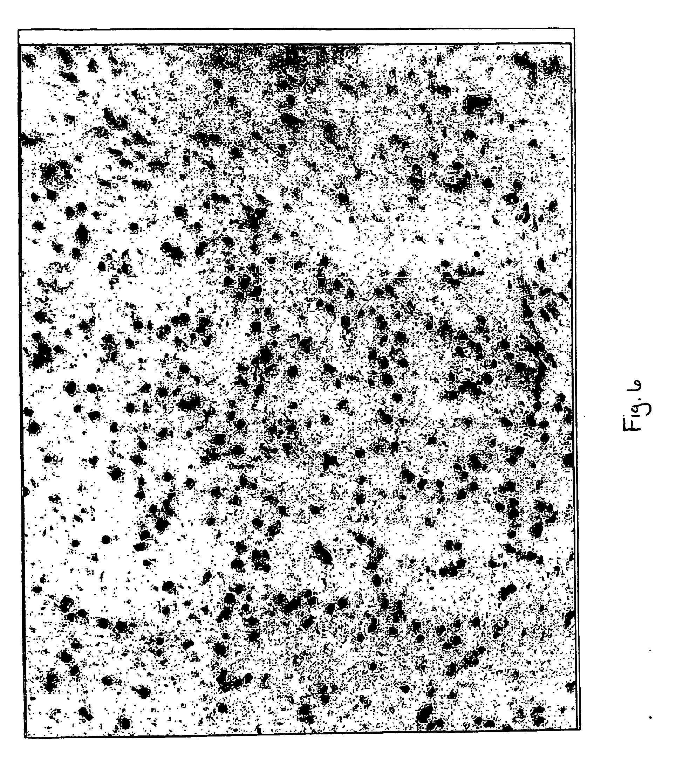

Tissues of the immune system were examined for the presence of non-mammalian GnRH receptors in their cells. The presence of non-mammalian GnRH receptors in the tissues of humans has not been previously described. This present investigation demonstrated in immune cells of mammalian tissues that non-mammalian GnRH receptors are produced in the mammals and that they are present in the immune system. See Fig. 6.

Human tissues from the thymus, spleen and lymph nodes were fixed and sectioned and plated by sections on glass slides. The human tissues on the glass slides were incubated with anti-GnRH II (1/100) for 1hour at RT. The tissues were then washed with phosphate buffered saline and anti-rabbit gamma globulin conjugated with biotin is incubated for 4 minutes at 55 C. The slide was rinsed in buffer followed by blocking of the endogenous peroxidase activity. Then streptavidin horse radish peroxidase was added and incubated for 4 minutes at 55 C. Stable diaminobenzidine (5 minutes at 55 C) was used to generate the signal. The slides were rinsed, mounted and read. The presence of GnRH II was localized via the DAB using microscopy. In the immune tissues examined, spleen, thymus and lymph node GnRH II was visualized. Tissues such as atrium and liver were negative.

### EXAMPLE X- Receptor Binding Activity

The receptor binding activity of non-mammalian GnRH (SEQ ID NO: 6 and SEQ ID NO: 7) and non-mammalian GnRH analogs (SEQ ID NO: 2 and SEQ ID NO: 4) of the present invention will be compared. There is a human non-mammalian GnRH receptor which is distinct from the GnRH I receptor at the pituitary. Prior GnRH I analogs have been designed to increase activity at the pituitary GnRH I receptor and stability in the circulation of individuals. These GnRH I analogs do not demonstrate potent binding activity at the immune system's GnRH II receptors as they do at the pituitary's GnRH I receptor. The present non-mammalian GnRH analogs (SEQ ID NO: 2 and SEQ ID NO: 4) have been designed to interact with preference at the immune system GnRH II receptors and not the GnRH I receptor. They have also been designed to limit degradation by the immune system enzymes, present in lymphatic circulation. Binding activity of the newly synthesized non-mammalian GnRH analogs (SEQ ID NO: 2 and SEQ ID NO: 4) has been studied in plasma.

The newly synthesized non-mammalian GnRH analogs (SEQ ID NO: 2 and SEQ ID NO: 4) and other commercially available analogs have been used in receptor binding studies in plasma and enzyme stability study described here. On the basis of these studies, the most receptor potent and most enzyme-stable analogs have been chosen for further biopotency studies. GnRH receptors have been purified from the fractions from immune system tissues. The purification procedure for the GnRH receptor utilized ethanol precipatation of the receptor and not the GnRH. The remaining non-mammalian GnRH binding assays activity using ¹²⁵I- D-Arg-GnRH II-Aza-Gly-NH₂¹²⁵ label and GnRH II have been performed. Receptors from two different tissues from the same type of immune system cells have been used to study each of these analogs. These data have enabled the inventor to predict the most potent non-mammalian GnRH analog structure for the non-mammalian GnRH receptor in the immune system, and assist in the design of even more potent analogs for the GnRH receptor. Bramley TA, McPhie CA, Menzies GS 1994 Human placental gonadotropin-releasing hormone (GnRH) binding sites: 111. Changes in GnRH binding levels with stage of gestation. Placenta 15:733-745.

In these studies, GnRH receptors have been purified from human immune system tissue after ethanol precipitation and extraction of GnRH in the supernatant. The binding affinity for the receptor free and containing supernants were compared for each GnRH II (SEQ ID NO: 6) or analog (SEQ ID NO: 2) have been compared. Each study has been done using two different human immune system tissues.

### EXAMPLE XI - Activity of Non-Mammalian GnRH or its Analogs on Immune System Tissues

Tissues of the immune system have been examined for the ability of their cells in vitro to respond to GnRH II (SEQ ID NO: 6) in culture medium. The media from cell cultures of immune system tissues have been examined for the release of cytokine into the medium after incubation with and without GnRH II analog (SEQ ID NO: 2).

Cell cultures of human leukocytes tissues have been prepared. These cells have been cultured in the presence and absence of non-mammalian GnRH (SEQ ID NO: 6 and SEQ ID NO: 7) and its analogs (SEQ ID NO: 2 and SEQ ID NO: 4) and GnRH I (SEQ ID NO: 5) and its analogs at varying doses. The release of cytokines into the medium have been determined and compared for each form of GnRH studied. GnRH II (SEQ ID NO: 6) and its analogs (SEQ ID NO: 2) had greater activity on immune systems cytokines and GnRH II analog (SEQ ID NO: 2) was the most active.

We studied the effect of cytokines primarily produced by T cells. We have found no effects of GnRH II (SEQ ID NO: 6), our GnRH II analog (SEQ ID NO: 2), GnRH I (SEQ ID NO: 5) and Buserelin (SEQ ID NO: 10) on Interferon γ (INFγ), IL-4, IL-8 and IL-10 on the low production of these cytokines by any of these peptides using this system. Concentrations of 2x10⁻⁹ to 2x10⁻⁷ M at 1, 3 and 20 hours were studied.

We have also studied cytokine produced primarily by B cells and macrophages. We observed that GnRH II analog (SEQ ID NO: 2) and Buserelin (SEQ ID NO: 10), a GnRH I agonist and a partial GnRH II agonist, inhibited Interleukin 12 (IL-12) after even one hour of treatment, which was still observed at 3 and 20 hours. The natural isoform of GnRH II (SEQ ID NO: 6) also effected an inhibition at 3 hours while GnRH I (SEQ ID NO: 5) increased IL-12 followed by a decrease at 20 hours using high dose of GnRH I (SEQ ID NO: 5). This is consistent with opposing activities followed by downregulation of the receptors with chronic high concentrations of the ligand.

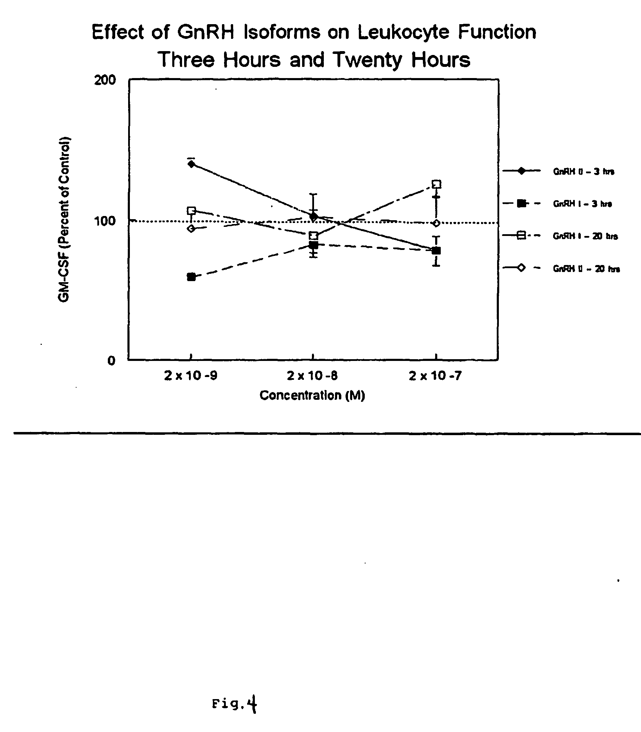

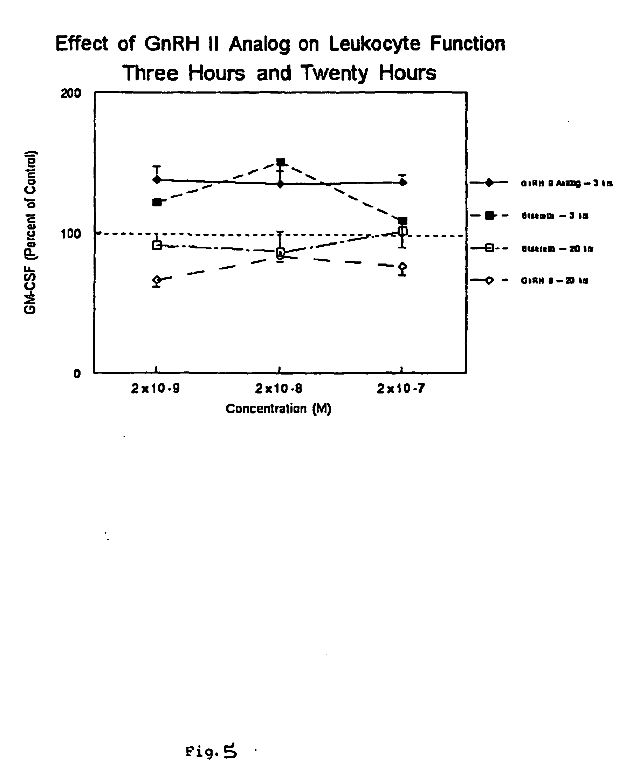

We have also demonstrated that GnRH II (SEQ ID NO: 6) at low dose is a potent stimulant of granulocyte macrophage colony stimulating factor, GM-CSF, while at high dose inhibits this cytokine as expected with down-regulation or the non-mammalian GnRH receptor. The activity is observed at 3 hrs but not as 20 hours due to the limited stability of GnRH in biological fluids. Fig. 4 shows the effect of GnRH isoforms I and II on leukocyte function, specifically granulocyte/macrophage colony stimulating factors (GM-CSF) release from Human leukocytes, at three hours and twenty hours. Using our GnRH II analog (SEQ ID NO: 2) an inhibition of GM-CSF was clearly apparent after 20 hours of exposure. Fig. 5 shows the effect of the GnRH II analog on leukocyte function, specifically granulocyte/macrophage colony stimulating factors (GM-CSF) release from human leukocytes, atthree hours and twenty hours. We compared these effects to that for GnRH I (SEQ ID NO: 5) and found essentially opposite results for GnRH II (SEQ ID NO: 6). We also studied Buserelin (SEQ ID NO: 10), GnRH I agonist and a partial GNRH) II agonist and observed a similar but lesser activity as our GnRH II analog (SEQ ID NO: 2).

## Claims

1. Use of a Chicken II GnRH decapeptide analog, having the sequence p-Glu-His-Trp-Ser-His-Xaa1-Trp-Tyr-Pro-Xaa2, capable of binding to GnRH receptors within the immune system and active in the presence of a post-proline peptidase or an endopeptidase, said analog comprising a D-amino acid substitution at position 6 and an ethylamide or aza-Gly-amide substitution at position 10, for the manufacture of a medicament for treating a condition of the immune system of a patient or animal, the condition selected from the group consisting of allergies and asthma, graft versus host disease, immune deficiency diseases, autoimmune disease, inflammation and tumour rejection, immune processes regulating implantation and pregnancy, endometrious, uterine fibroids, immune involved disease, acutely stimulate or choronically inhibit over-activity of the immune system.

2. Use of the Chicken II GnRH decapeptide analog of claim 1 wherein the Chicken II GnRH analog is further defined as D-Arg(6)-Chicken II GnRH-aza-Gly(10)-amide having a sequence as defined in SEQ ID NO: 2 (p-Glu-His-Trp-Ser-His-D-Arg-Trp-Tyr-Pro-aza-Gly-NH₂).

3. Use of the Chicken II GnRH decapeptide analog of claim 2 wherein the Chicken II GnRH has been derived from a DNA sequence of SEQ ID NO: 1 (CAG CAC TGG TCT CAT GGC TGG TAT CCT GGA).

4. Use of the Chicken II GnRH decapeptide analog of claim 1 wherein the Chicken II GnRH analog is further defined as comprising a D-Arg, a D-Leu, D-tBu-serine, or a D-Trp substitution at position 6 and an aza-Gly amide or an ethylamide at position 10.

5. Use of a polypeptide according to claim 1 having the amino acid sequence set forth in SEQ ID NO: 2, or SEQ ID NO: 2 with at least one conservative amino acid substitution, or mimetic functional equivalent thereof, for the manufacture of a medicament for treating a condition of the immune system of a patient or animal, the condition selected from the group consisting of allergies and asthma, graft versus host disease, immune deficiency diseases, autoimmune disease, inflammation and tumour rejection, immune processes regulating implantation and pregnancy, endometrious, uterine fibroids, immune involved disease, acutely stimulate or choronically inhibit over-activity of the immune system.

6. Use of an antibody to the polypeptide according to claim 1 with SEQ ID NO: 2, or SEQ ID NO: 2 with at least one conservative amino acid substitution, for the manufacture of a medicament for treating a condition of the immune system of a patient or animal, the condition selected from the group consisting of allergies and asthma, graft versus host disease, immune deficiency diseases, autoimmune disease, inflammation and tumour rejection, immune processes regulating implantation and pregnancy, endometrious, uterine fibroids, immune involved disease, acutely stimulate or choronically inhibit over-activity of the immune system.

7. Use of an expression control vector comprising an expression control sequence that directs production of a transcript that hybridizes under physiological conditions to the nucleic acid sequence or the complement thereof for the purified mammalian polypeptide according to claim 1, capable of binding to a mimetic functional equivalent of SEQ ID No: 2, or SEQ ID NO: 2 with at least one conservative amino acid substitution, for the manufacture of a medicament for treating a condition of the immune system of a patient or animal, the condition selected from the group consisting of allergies and asthma, graft versus host disease, immune deficiency diseases, autoimmune disease, inflammation and tumour rejection, immune processes regulating implantation and pregnancy, endometrious, uterine fibroids, immune involved disease, acutely stimulate or choronically inhibit over-activity of the immune system.

8. Use of a polypeptide having the amino acid sequence set forth in SEQ ID NO: 6, or SEQ ID NO: 6 with at least one conservative amino acid substitution, or mimetic equivalent thereof, for the manufacture of a medicament for treating a condition of the immune system of a patient or animal, the condition selected from the group consisting of allergies and asthma, graft versus host disease, immune deficiency diseases, autoimmune disease, inflammation and tumour rejection, immune processes regulating implantation and pregnancy, endometrious, uterine fibroids, immune involved disease, acutely stimulate or choronically inhibit over-activity of the immune system.

9. Use of an antibody to the polypeptide with SEQ ID NO: 6, or SEQ ID NO: 6 with at least one conservative amino acid substitution, or mimetic functional equivalent thereof, for the manufacture of a medicament for treating a condition of the immune system of a patient or animal, the condition selected from the group consisting of allergies and asthma, graft versus host disease, immune deficiency diseases, autoimmune disease, inflammation and tumour rejection, immune processes regulating implantation and pregnancy, endometrious, uterine fibroids, immune involved disease, acutely stimulate or choronically inhibit over-activity of the immune system.

## Patentansprüche

1. Verwendung eines Hühner-II-GnRH-Decapeptid-Analogons mit der Sequenz p-Glu-His-Trp-Ser-His-Xaa1-Trp-Tyr-Pro-Xaa2, das imstande ist, an GnRH-Rezeptoren im Immunsystem zu binden und in Gegenwart einer post-Prolin-Peptidase oder einer Endopeptidase aktiv ist, wobei das Analogon eine D-Aminosäuresubstitution an Position 6 und eine Ethylamid- oder aza-Gly-Amidsubstitution an Position 10 umfasst, zur Herstellung eines Arzneimittels zur Behandlung einer Erkrankung des Immunsystems eines Patienten oder Tieres, wobei die Erkrankung ausgewählt ist aus der Gruppe, bestehend aus Allergien und Asthma, Transplantat-gegen-Wirt-Erkrankung, Immundefekterkrankungen, Autoimmunerkrankung, Entzündung und Tumorabstoßung, Immunvorgänge, die Implantation und Schwangerschaft regulieren, Endometriose, Gebärmutterfibrome, das Immunsystem betreffende Erkrankung, akutes Stimulieren oder chronisches Verhindern einer Überaktivität des Immunsystems.

2. Verwendung des Hühner-II-GnRH-Decapeptid-Analogons nach Anspruch 1, wobei das Hühner-II-GnRH-Analogon weiterhin definiert ist als D-Arg(6)-Hühner-II-GnRH-aza-Gly(10)-amid mit einer Sequenz, wie in SEQ ID NO: 2 (p-Glu-His-Trp-Ser-His-D-Arg-Trp-Tyr-Pro-aza-Gly-NH₂) definiert.

3. Verwendung des Hühner-II-GnRH-Decapeptid-Analogons nach Anspruch 2, wobei das Hühner-II-GnRH von einer DNA-Sequenz von SEQ ID NO: 1 (CAG CAC TGG TCT CAT GGC TGG TAT CCT GGA) abgeleitet ist.

4. Verwendung des Hühner-II-GnRH-Decapeptid-Analogons nach Anspruch 1, wobei das Hühner-II-GnRH-Analogon weiterhin definiert ist als eine D-Arg-, eine D-Leu-, D-tBu-Serin oder eine D-Trp-Substitution an Position 6 und ein aza-Gly-Amid oder ein Ethylamid an Position 10 umfassend.

5. Verwendung eines Polypeptids nach Anspruch 1 mit der in SEQ ID NO: 2 dargelegten Aminosäuresequenz, oder SEQ ID NO: 2 mit zumindest einer konservativen Aminosäuresubstitution oder ein mimetisches funktionelles Äquivalent derselben zur Herstellung eines Arzneimittels zur Behandlung einer Erkrankung des Immunsystems eines Patienten oder Tieres, wobei die Erkrankung ausgewählt ist aus der Gruppe, bestehend aus Allergien und Asthma, Transplantat-gegen-Wirt-Erkrankung, Immundefekterkrankungen, Autoimmunerkrankung, Entzündung und Tumorabstoßung, Immunvorgänge, die Implantation und Schwangerschaft regulieren, Endometriose, Gebärmutterfibrome, das Immunsystem betreffende Erkrankung, akutes Stimulieren oder chronisches Verhindern einer Überaktivität des Immunsystems.

6. Verwendung eines Antikörpers bei dem Polypeptid nach Anspruch 1 mit SEQ ID NO: 2, oder SEQ ID NO: 2 mit zumindest einer konservativen Aminosäuresubstitution zur Herstellung eines Arzneimittels zur Behandlung einer Erkrankung des Immunsystems eines Patienten oder Tieres, wobei die Erkrankung ausgewählt ist aus der Gruppe, bestehend aus Allergien und Asthma, Transplantat-gegen-Wirt-Erkrankung, Immundefekterkrankungen, Autoimmunerkrankung, Entzündung und Tumorabstoßung, Immunvorgänge, die Implantation und Schwangerschaft regulieren, Endometriose, Gebärmutterfibrome, das Immunsystem betreffende Erkrankung, akutes Stimulieren oder chronisches Verhindern einer Überaktivität des Immunsystems.

7. Verwendung eines Expressionskontrollvektors, umfassend eine Expressionskontrollsequenz, die eine Herstellung eines Transkripts leitet, das unter physiologischen Bedingungen zur Nukleinsäuresequenz oder deren Komplement für das gereinigte Säugetierpolypeptid nach Anspruch 1 hybridisiert, das imstande ist, an ein mimetisches funktionelles Äquivalent von SEQ ID No: 2 zu binden, oder SEQ ID NO: 2 mit zumindest einer konservativen Aminosäuresubstitution zur Herstellung eines Arzneimittels zur Behandlung einer Erkrankung des Immunsystems eines Patienten oder Tieres, wobei die Erkrankung ausgewählt ist aus der Gruppe, bestehend aus Allergien und Asthma, Transplantat-gegen-Wirt-Erkrankung, Immundefekterkrankungen, Autoimmunerkrankung, Entzündung und Tumorabstoßung, Immunvorgänge, die Implantation und Schwangerschaft regulieren, Endometriose, Gebärmutterfibrome, das Immunsystem betreffende Erkrankung, akutes Stimulieren oder chronisches Verhindern einer Überaktivität des Immunsystems.

8. Verwendung eines Polypeptids mit der in SEQ ID NO: 6 dargelegten Aminosäuresequenz, oder SEQ ID NO: 6 mit zumindest einer konservativen Aminosäuresubstitution oder deren mimetisches Äquivalent zur Herstellung eines Arzneimittels zur Behandlung einer Erkrankung des Immunsystems eines Patienten oder Tieres, wobei die Erkrankung ausgewählt ist aus der Gruppe, bestehend aus Allergien und Asthma, Transplantat-gegen-Wirt-Erkrankung, Immundefekterkrankungen, Autoimmunerkrankung, Entzündung und Tumorabstoßung, Immunvorgänge, die Implantation und Schwangerschaft regulieren, Endometriose, Gebärmutterfibrome, das Immunsystem betreffende Erkrankung, akutes Stimulieren oder chronisches Verhindern einer Überaktivität des Immunsystems.

9. Verwendung eines Antikörpers bei dem Polypeptid mit SEQ ID NO: 6, oder SEQ ID NO: 6 mit zumindest einer konservativen Aminosäuresubstitution oder mimetisches funktionelles Äquivalent derselben zur Herstellung eines Arzneimittels zur Behandlung einer Erkrankung des Immunsystems eines Patienten oder Tieres, wobei die Erkrankung ausgewählt ist aus der Gruppe, bestehend aus Allergien und Asthma, Transplantat-gegen-Wirt-Erkrankung, Immundefekterkrankungen, Autoimmunerkrankung, Entzündung und Tumorabstoßung, Immunvorgänge, die Implantation und Schwangerschaft regulieren, Endometriose, Gebärmutterfibrome, das Immunsystem betreffende Erkrankung, akutes Stimulieren oder chronisches Verhindern einer Überaktivität des Immunsystems.

## Revendications

1. Utilisation d'un décapeptide analogue GnRH II de poulet, ayant la séquence p-Glu-His-Trp-Ser-His-Xaa1-Trp-Tyr-Pro-Xaa2, capable de se lier aux récepteurs de GnRH dans le système immunitaire et actif en présence d'une peptidase post-proline ou d'une endopeptidase, l'analogue comprenant une substitution de D-aminoacide à la position 6 et une substitution d'éthylamide ou d'amide aza-Gly à la position 10, pour la fabrication d'un médicament destiné au traitement d'un trouble du système immunitaire d'une personne ou d'un animal, le trouble étant sélectionné parmi les suivants : allergies et asthme, maladie du greffon contre l'hôte, maladies liées à une déficience immunitaire, maladie auto-immune, inflammation et rejet tumoral, processus immunitaires régulant l'implantation et la grossesse, maladie de l'endomètre, fibromes utérins, maladies liées au système immunitaire, stimulation aiguë ou inhibition chronique de la suractivité du système immunitaire.

2. Utilisation du décapeptide analogue GnRH II de poulet de la revendication 1, l'analogue GnRH II de poulet étant également défini en tant qu'amide aza-Gly(10) GnRH II de poulet D-Arg(6) ayant la séquence définie dans SEQ ID NO: 2 (p-Glu-His-Trp-Ser-His-D-Arg-Trp-Tyr-Pro-aza-Gly-NH₂).

3. Utilisation du décapeptide analogue GnRH II de poulet de la revendication 2, l'analogue GnRH II de poulet provenant d'une séquence d'ADN de SEQ ID NO: 1 (CAG CAC TGG TCT CAT GGC TGG TAT CCT GGA).

4. Utilisation du décapeptide analogue GnRH II de poulet de la revendication 1, dans lequel l'analogue GnRH II de poulet est également défini comme contenant une substitution de D-Arg, D-Leu, sérine D-tBu ou D-Trp à la position 6 et un amide aza-Gly ou un éthylamide à la position 10.

5. Utilisation d'un polypeptide selon la revendication 1, ayant la séquence d'acides aminés indiquée dans SEQ ID NO: 2, ou SEQ ID NO: 2 avec au moins une substitution d'acide aminé conservatoire ou un équivalent fonctionnel mimétique, pour la fabrication d'un médicament destiné au traitement d'un trouble du système immunitaire d'une personne ou d'un animal, le trouble étant sélectionné parmi les suivants : allergies et asthme, maladie du greffon contre l'hôte, maladies liées à une déficience immunitaire, maladie auto-immune, inflammation et rejet tumoral, processus immunitaires régulant l'implantation et la grossesse, maladie de l'endomètre, fibromes utérins, maladies liées au système immunitaire, stimulation aiguë ou inhibition chronique de la suractivité du système immunitaire.

6. Utilisation d'un anticorps anti-polypeptide ayant la séquence de SEQ ID NO: 2, ou SEQ ID NO: 2 avec au moins une substitution d'acide aminé conservatoire, pour la fabrication d'un médicament destiné au traitement d'un trouble du système immunitaire d'une personne ou d'un animal, le trouble étant sélectionné parmi les suivants : allergies et asthme, maladie du greffon contre l'hôte, maladies liées à une déficience immunitaire, maladie auto-immune, inflammation et rejet tumoral, processus immunitaires régulant l'implantation et la grossesse, maladie de l'endomètre, fibromes utérins, maladies liées au système immunitaire, stimulation aiguë ou inhibition chronique de la suractivité du système immunitaire.

7. Utilisation d'un vecteur de contrôle d'expression comprenant une séquence de contrôle d'expression dirigeant la production d'une transcription qui s'hybride dans certaines conditions physiologiques à la séquence d'acide nucléique ou son complément pour le polypeptide de mammifère purifié conformément à la revendication 1, capable de se lier à un équivalent fonctionnel mimétique de SEQ ID No: 2, ou SEQ ID NO: 2 avec au moins une substitution d'acide aminé conservatoire, pour la fabrication d'un médicament destiné au traitement d'un trouble du système immunitaire d'une personne ou d'un animal, le trouble étant sélectionné parmi les suivants : allergies et asthme, maladie du greffon contre l'hôte, maladies liées à une déficience immunitaire, maladie auto-immune, inflammation et rejet tumoral, processus immunitaires régulant l'implantation et la grossesse, maladie de l'endomètre, fibromes utérins, maladies liées au système immunitaire, stimulation aiguë ou inhibition chronique de la suractivité du système immunitaire.

8. Utilisation d'un polypeptide ayant la séquence d'acide aminé indiquée dans SEQ ID NO: 6, ou SEQ ID NO: 6 avec au moins une substitution d'acide aminé conservatoire ou un équivalent mimétique, pour la fabrication d'un médicament destiné au traitement d'un trouble du système immunitaire d'une personne ou d'un animal, le trouble étant sélectionné parmi les suivants : allergies et asthme, maladie du greffon contre l'hôte, maladies liées à une déficience immunitaire, maladie auto-immune, inflammation et rejet tumoral, processus immunitaires régulant l'implantation et la grossesse, maladie de l'endomètre, fibromes utérins, maladies liées au système immunitaire, stimulation aiguë ou inhibition chronique de la suractivité du système immunitaire.

9. Utilisation d'un anticorps anti-polypeptide ayant la séquence de SEQ ID NO: 6, ou SEQ ID NO: 6 avec au moins une substitution d'acide aminé conservatoire ou un équivalent fonctionnel mimétique, pour la fabrication d'un médicament destiné au traitement d'un trouble du système immunitaire d'une personne ou d'un animal, le trouble étant sélectionné parmi les suivants : allergies et asthme, maladie du greffon contre l'hôte, maladies liées à une déficience immunitaire, maladie auto-immune, inflammation et rejet tumoral, processus immunitaires régulant l'implantation et la grossesse, maladie de l'endomètre, fibromes utérins, maladies liées au système immunitaire, stimulation aiguë ou inhibition chronique de la suractivité du système immunitaire.
